(19) Europäisches Patentamt
European Patent Office
Office européen des brevets

(11) **EP 4 008 413 A1**

(12) **EUROPEAN PATENT APPLICATION**
published in accordance with Art. 153(4) EPC

(43) Date of publication:
**08.06.2022 Bulletin 2022/23**

(21) Application number: **20849150.6**

(22) Date of filing: **31.07.2020**

(51) International Patent Classification (IPC):
*A63B 71/06* (2006.01)    *G16H 20/30* (2018.01)
*A61B 5/00* (2006.01)    *A61B 5/11* (2006.01)
*A61B 5/145* (2006.01)    *A61B 5/22* (2006.01)

(52) Cooperative Patent Classification (CPC):
**A61B 5/00; A61B 5/11; A61B 5/145; A61B 5/22;
A63B 71/06; G16H 20/30**

(86) International application number:
**PCT/JP2020/029499**

(87) International publication number:
**WO 2021/024949 (11.02.2021 Gazette 2021/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **02.08.2019 JP 2019142978**

(71) Applicant: **Astellas Pharma Inc.
Chuo-ku
Tokyo 103-8411 (JP)**

(72) Inventor: **KANAYAMA, Motohiro
Tokyo 103-8411 (JP)**

(74) Representative: **Gunzelmann, Rainer
Wuesthoff & Wuesthoff
Patentanwälte PartG mbB
Schweigerstraße 2
81541 München (DE)**

(54) **EXERCISE MANAGEMENT SYSTEM, SERVER SYSTEM, TERMINAL DEVICE, AND EXERCISE MANAGEMENT METHOD**

(57)    The exercise management system includes a storage section (170) for storing user personal information; a processing section (100) for determining a resistance exercise content and an aerobic exercise content based on the user personal information; and an acquisition section (communication section 192) for acquiring motion information indicating motion of the user when exercise instructions are given based on the resistance exercise content, and vital information indicating vitals of the user when the exercise instructions are given based on the aerobic exercise content. Based on the motion information and the vital information, the processing section (100) performs a process of presenting performance result information of resistance exercise and aerobic exercise performed by the user on a user terminal device (TMA), or another terminal device (TMB).

FIG. 3

## Description

TECHNICAL FIELD

The present invention relates to an exercise management system, a server system, a terminal device, and an exercise management method, and the like.

BACKGROUND ART

A method of supporting exercise therapy for patients with diabetes and the like by detecting vital information, such as the user's motions and the heart rate, during exercise has been previously known. For example, Patent Literature 1 discloses a method to assist users in exercising by displaying exercise information to help the users to achieve a specified exercise intensity. Patent Literature 1 also discloses a method of using a pacemaker having virtual exercise ability as exercise information.

CITATION LIST

PATENT LITERATURE

Patent Literature 1: Japanese Laid-Open Patent Application Publication No. 2018-201905

SUMMARY OF INVENTION

TECHNICAL PROBLEM

In exercise therapy, it is effective to combine resistance exercise, which applies loads to the muscles, with aerobic exercise, which is a relatively light-loaded exercise is continued for a long time. Although previously-known techniques such as Patent Literature 1 disclose a method for supporting users by setting target heart rate, and the like, they do not disclose combining resistance exercise with aerobic exercise.

According to some of the embodiments disclosed herein, it is possible to provide an exercise management system, a server system, a terminal device, and an exercise management method, and the like, by which an effective exercise combining resistance exercise and aerobic exercise can be easily performed.

SOLUTION TO PROBLEM

One of the embodiments of the present disclosure is an exercise management system for managing exercises of a user, who is a type-2 diabetic patient or a type-2 pre-diabetic patient, comprising a storage section for storing user personal information of the user; a processing section for determining, based on the user personal information, a resistance exercise content, which is information to specify a content of resistance exercise performed by the user, and an aerobic exercise content, which is information to specify a content of aerobic exercise performed by the user; and an acquisition section for acquiring motion information indicating the motion of the user when exercise instructions are given based on the resistance exercise content, and vital information indicating vitals of the user when the exercise instructions are given based on the aerobic exercise content, wherein the processing section performs, based on the motion information and the vital information, a process of presenting performance result information indicating performance results of the resistance exercise and the aerobic exercise performed by the user on a user terminal device used by the user, or another terminal device.

In some embodiments of the present disclosure, it is possible to determine the contents of resistance exercise and aerobic exercise performed by the user based on the user personal information. As a result, even users who do not have specialized knowledge of exercise can easily perform exercises appropriate for the user. Also, by presenting the performance result information based on the motion information and the vital information at the time of the exercise, it is possible to present exercise performance results to the user or medical staff and the like in charge of the user, in a manner in which they easily understand the results.

In some embodiments of the present disclosure, the user personal information may include at least one of the gender, age, constitutional information, and physical measurement information of the user.

As a result, it is possible to appropriately determine the exercise content considering the gender and the like of the user.

In some embodiments of the present disclosure, the user personal information may include the performance result information of at least one of the resistance exercise and the aerobic exercise performed by the user in the past.

As a result, it is possible to appropriately determine the exercise content considering the performance results of exercise in the past.

In some embodiment of the present disclosure, the resistance exercise content and the aerobic exercise content performed in a single exercise determined by the processing section may be an exercise program including the resistance exercise content corresponding to a type of the resistance exercise selected from among a plurality of types of the resistance exercises, and the aerobic exercise content corresponding to a type of the aerobic exercise selected from among a plurality of types of the aerobic exercises.

As a result, it is possible to make users to perform a single unit of exercises by combining appropriate exercises from among a plurality of types of resistance exercises and a plurality of types of aerobic exercises.

In some embodiments of the present disclosure, the processing section may determine a plurality of types of the exercise programs to be performed by the user in a given exercise period based on the user personal information.

As a result, it is possible to appropriately determine information to make users continuously perform exercises.

In some embodiments of the present disclosure, the processing section may determine the resistance exercise content and the aerobic exercise content based on input from the another terminal device.

As a result, the medical staff, the system administrators, and the like can be involved in the determination of exercise programs.

In some embodiments of the present disclosure, the processing section may determine the resistance exercise content and the aerobic exercise content based on an exercise period or a number of times of exercise when the exercise period or the number of times of exercise performed by the user is input from the user terminal device or the another terminal device.

As a result, it is possible to appropriately determine the exercise content considering the exercise period or the number of times of the exercise.

In some embodiments of the present disclosure, the storage section may store table data in which the resistance exercise content and the aerobic exercise content are associated with each other.

As a result, it is possible to easily determine the exercise program based on the table data.

In some embodiments of the present disclosure, when one of the resistance exercise content and the aerobic exercise content is determined, the processing section may determine the other one of the resistance exercise content and the aerobic exercise content based on the determined resistance exercise content or aerobic exercise content.

As a result, it is possible to determine one of the resistance exercise content and the aerobic exercise content according to the other. For example, if there is an exercise content(s) preferentially performed, it is possible to determine the exercise program according to the exercise content.

In some embodiments of the present disclosure, the processing section may determine a prize to be given to the user based on the performance result information of the user in response to the exercise instructions based on the resistance exercise content and the aerobic exercise content.

As a result, by thus giving a prize according to the performance results, it is possible to improve the user's motivation to exercise.

In some embodiments of the present disclosure, the processing section may perform a process of setting a friend user, who is another user or a virtual user who performs exercises corresponding to the resistance exercise content and the aerobic exercise content for which the user is instructed to perform.

As a result, it is possible to improve users' motivation to exercise by using the friend user.

In some embodiments of the present disclosure, the processing section may perform a process of presenting information to encourage the user to exercise based on a difference between the performance result information of the user and the performance result information of the friend user.

As a result, it is possible to improve users' motivation to exercise by using the difference from the friend user.

In some embodiments of the present disclosure, when the degree of achievement for the exercise instructions is determined to be higher than a given standard based on the performance result information of the user, the processing section may perform a process of skipping presentation of the information to encourage the user to exercise based on the difference, or performs a process of presenting information suggesting that the user can reduce exercises based on the difference.

As a result, it is possible to prevent the user from performing excessive exercise by suppressing the encouragement of exercise or by suppressing the exercise.

In some embodiments of the present disclosure, the processing section may acquire reference data indicating standard performance result information of the resistance exercise content and the aerobic exercise content for which the user is instructed to perform, and may perform a process of presenting information to encourage the user to exercise or a process of presenting information that suggests that the user can reduce the exercise based on the difference between the performance result information of the user and the reference data.

As a result, it is possible to improve user's motivation to exercise or suppress excessive exercise by using the difference from the standard performance result information.

In some embodiments of the present disclosure, the processing section may present a first performance result information obtained based on the motion information, the vital information and a first determination criteria on the user terminal device, and present a second performance result information obtained based on the motion information, the vital information, and a second determination criteria, which is different from the first determination criteria, on the another terminal device.

As a result, it is possible to present different performance results on the user terminal device and another the terminal device even when the same the motion information and the vital information are used.

In some embodiments of the present disclosure, a motion detection section for detecting the motion information and a vital detection section for detecting the vital information may further be provided, and the motion detection section and the vital detection section may be provided in a single wearable terminal device attached to the user.

As a result, it is possible to reduce the burden on the user to wear the device and easily perform and manage the resistance exercise and the aerobic exercise.

Another embodiment of the present disclosure relates to a server system for managing exercises of a user, who is a type-2 diabetic patient or a type-2 pre-diabetic patient, comprising a storage section for storing user personal information of the user; a processing section for determining, based on the user personal information, a resistance exercise content, which is information to specify a content of resistance exercise performed by the user, and an aerobic exercise content, which is information to specify a content of aerobic exercise performed by the user; and an acquisition section for acquiring motion information indicating the motion of the user when exercise instructions are given based on the resistance exercise content, and vital information indicating vitals of the user when the exercise instructions are given based on the aerobic exercise content, wherein the processing section performs, based on the motion information and the vital information, a process of presenting performance result information indicating performance results of the resistance exercise and the aerobic exercise performed by the user on a user terminal device used by the user, or another terminal device.

Still another embodiment of the present disclosure relates to a terminal device for use in exercise management of a user, who is a type-2 diabetic patient or a type-2 pre-diabetic patient, the terminal device comprising: a processing section for performing a first acquisition process to acquire a resistance exercise content and an aerobic exercise content determined based on user personal information of the user; an instruction process to give exercise instructions to the user based on the resistance exercise content and the aerobic exercise content; and a second acquisition process to acquire motion information indicating motion of the user when the exercise instructions are given based on the resistance exercise content, and vital information indicating vitals of the user when the exercise instructions are given based on the aerobic exercise content; and a presentation section for presenting performance result information indicating performance results of resistance exercise and aerobic exercise performed by the user based on the motion information and the vital information.

Yet another embodiment of the present disclosure relates to an exercise management method for managing exercises of a user, who is a type-2 diabetic patient or a type-2 pre-diabetic patient, the exercise management method comprising: performing a process of acquiring user personal information of the user; performing a process of determining, based on the user personal information, a resistance exercise content, which is information to specify a content of resistance exercise performed by the user, and an aerobic exercise content, which is information to specify a content of aerobic exercise performed by the user; performing a process of acquiring motion information indicating motion of the user when exercise instructions are given based on the resistance exercise content, and vital information indicating vitals of the user when the exercise instructions are given based on the aerobic exercise content; and based on the motion information and the vital information, performing a process of presenting performance result information indicating performance results of the resistance exercise and the aerobic exercise performed by the user on a user terminal device used by the user, or another terminal device.

BRIEF DESCRIPTION OF DRAWINGS

FIGS. 1A to 1E are explanatory views of a hardware device for implementing an exercise management system of the present embodiment.
FIG. 2 is a configuration example of an exercise management system.
FIG. 3 is a specific explanatory view of a hardware device for implementing an exercise management system.
FIG. 4 illustrates an example of user personal information.
FIG. 5 illustrates an example of resistance exercise.
FIG. 6 illustrates an example of resistance exercise information.
FIG. 7 illustrates an example of resistance exercise information.
FIG. 8 illustrates an example of aerobic exercise information.
FIG. 9 illustrates an example of table data of an exercise program.
FIG. 10 is a flowchart explaining a registration process.
FIG. 11 is a flowchart explaining processes during exercise.
FIG. 12 is an explanatory view of a process of counting the number of times of motions.
FIGS. 13A to 13C illustrate depth determination and form determination based on motion information.
FIG. 14 is an example of heart rate information as vital information.
FIGS. 15A and 15B are explanatory views of a plurality of game effects that can be associated with a single resistance exercise.
FIGS. 16A and 16B are explanatory views of a plurality of game effects that can be associated with a single resistance exercise.
FIG. 17 is an explanatory view of a game cycle.
FIG. 18 illustrates an example of a game screen.
FIG. 19 illustrates an example of a game screen.
FIG. 20 illustrates an example of a game screen.
FIG. 21 illustrates examples of prizes in the first and second games.
FIG. 22 is a flowchart explaining a process of setting a rest period.
FIG. 23 is a flowchart explaining a suspension determination process.

FIG. 24 illustrates an example of a screen displayed on another terminal device.

FIG. 25 illustrates an example of a screen displayed on a user terminal device.

FIG. 26 illustrates an example of a screen displaying a difference in performance result information.

FIG. 27 illustrates an example of a screen displaying a difference in performance result information.

DESCRIPTION OF EMBODIMENTS

Exemplary embodiments are described below. Note that the following exemplary embodiments do not in any way limit the scope of the content defined by the claims laid out herein. Note also that all of the elements described in the present embodiment should not necessarily be taken as essential elements.

1. System Configuration Example

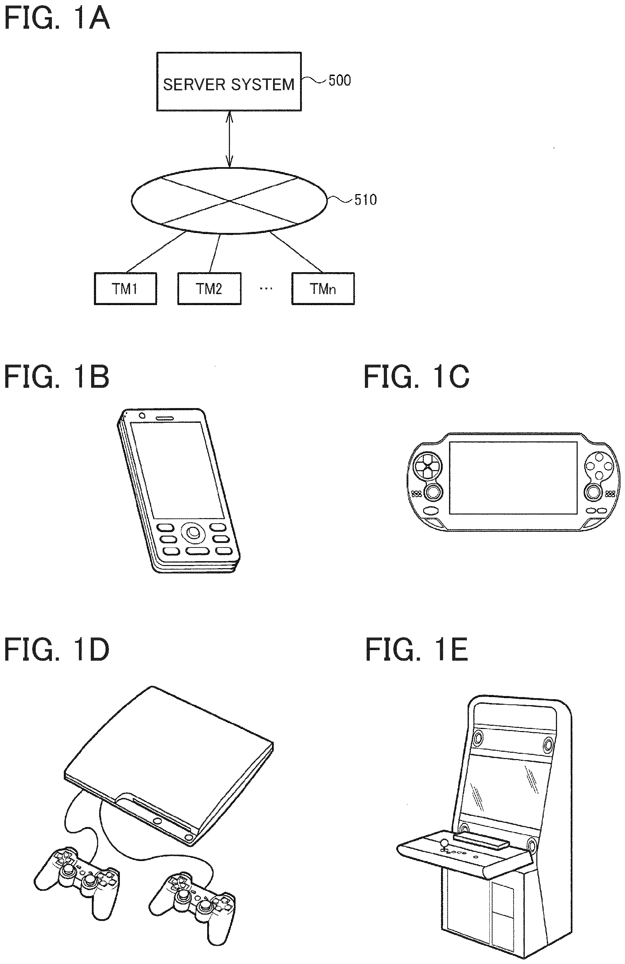

First, a hardware device for implementing an exercise management system according to the present embodiment is described below with reference to FIGS. 1A to IE.

In FIG. 1A, a server system 500 (information processing system) is communicably connected to terminal devices TM1 to TMn via a network 510. For example, the server system 500 is a server of a client-server system, and the terminal devices TM1 to TMn are of clients. The exercise management system according to the present embodiment and processing thereof may be implemented by the server system 500, or by the terminal devices TM1 to TMn. The exercise management system according to the present embodiment and processing thereof may also be implemented by a distributed process by the server system 500 and the terminal devices TM1 to TMn.

The exercise management system and the processing of the present embodiment can also be implemented by a method for blockchain. For example, each process of the exercise management system of the present embodiment may be executed using a program called a smart contract that can be executed by Ethereum. In this case, the terminal devices TM1 to TMn are connected by means of peer-to-peer. Various types of information such as game information communicated between the terminal devices TM1 to TMn are transferred using blockchain. Hereinafter, each of the terminal devices TM1 to TMn is described as a terminal device TM as necessary.

The server system 500 can be implemented by, for example, one or a plurality of servers (a management server, a game server, a charging server, a service providing server, a content distribution server, an authentication server, a database server, a communication server, and the like). As described later, the exercise management system of the present embodiment may perform a game process. The server system 500 can provide various services for operating a community website, an online game, or the like, and is capable of managing data necessary for performing the game and distributing a client program, various types of data, and the like. This enables, for example, a terminal device TM serving as a user terminal to access the server system 500 to use a social networking service (SNS) or the like, thereby enabling it to play a network game such as an online game, a social game, or a consumer game provided by the server system 500.

The network 510 (distribution network, communication line) is a communication channel using, for example, the Internet, wireless LAN, or the like. The network 510 may include a communication network such as a telecommunication network, a cable network, or a wireless LAN, in addition to an exclusive line (exclusive cable) for direct connection or a LAN by means of Ethernet (registered trademark) or the like. The communication method may be wired or wireless.

The terminal device TM (user terminal) is, for example, a terminal having a net connection function (Internet connection function). Examples of the terminal device TM include various devices such as a portable communication terminal (a smartphone, a mobile phone) illustrated in FIG. 1B, a portable game device shown in FIG. 1C, a consumer game device (stationary type) shown in FIG. 1D, an arcade game apparatus shown in FIG. IE, or the like. Alternatively, an information processing device, such as a personal computer (PC) or tablet computer, may be used as the terminal device TM. Alternatively, a wearable device (an HMD, a watch-type device, or the like) worn on a part such as a head or an arm of the user may be used as the terminal device TM.

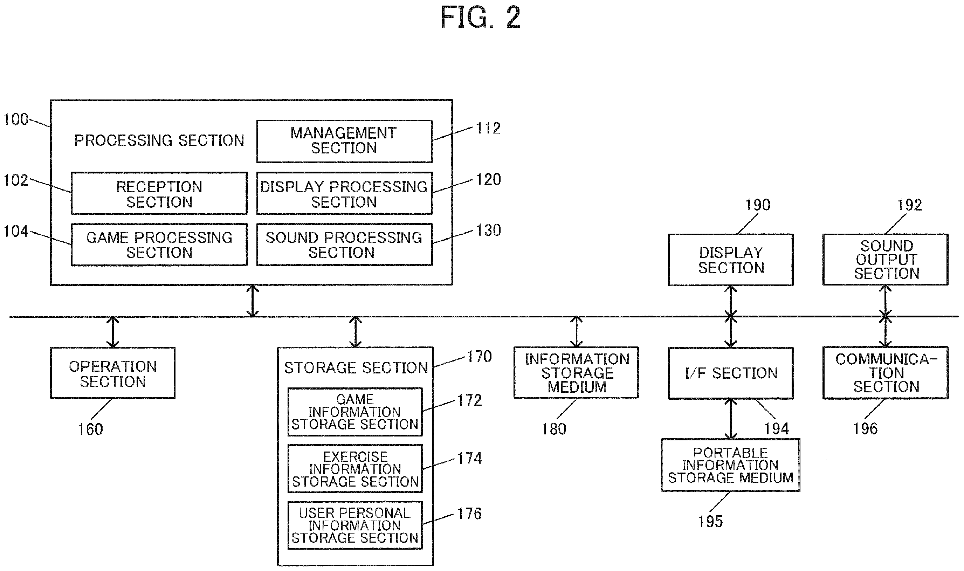

FIG. 2 illustrates a configuration example of an exercise management system of the present embodiment. The configuration of the exercise management system is not limited to that shown in FIG. 2, and can be modified in various ways including omitting some of its components (sections) or adding another component.

The exercise management system includes a processing section 100, an operation section 160, a storage section 170, a display section 190, a sound output section 192, an I/F section 194, and a communication section 196.

The processing section 100 (processor) performs a reception process, a game process, a management process, a display process, a sound process, or the like based on various types of information, programs, operation information, and the like stored in the storage section 170 (database).

The processes (functions) according to the present embodiment performed by sections of the processing section 100 can be implemented by a processor (processor including hardware). For example, the processes according to the present embodiment can be implemented by a processor that operates based on information such as a program and a memory that stores the information such as the program. For example, the processor may implement the functions of the sections in discrete hardware or in integrated hardware. For example, the processor may include hardware, and the hardware may include at least one of a circuit that processes a digital signal and a circuit that processes an analog signal. For example, the processor may include one or a plurality of circuit devices (such as an IC) or one or a plurality of circuit elements (such as a resistor or a capacitor) mounted on a circuit board. For example, the processor may be a central processing unit (CPU). However, the processor is not limited to the CPU, and various processors such as a graphics processing unit (GPU) or a digital signal processor (DSP) may be used. The processor may be a hardware circuit such as an ASIC. The processor may include an amplifier circuit, a filter circuit, or the like that processes an analog signal. The memory (storage section 170) may be a semiconductor memory such as a SRAM or DRAM, or may be a register. Furthermore, the memory may be a magnetic storage device such as a hard disk device (HDD) or may be an optical storage device such as an optical disc device. For example, the memory stores therein a computer-readable command, and the processes (functions) of the sections of the processing section 100 are implemented with the processor executing the command. This command may be a set of commands forming a program, or may be a command for instructing an operation to a hardware circuit of the processor.

The processing section 100 includes a reception section 102, a game processing section 104, a management section 112, a display processing section 120, and a sound processing section 130.

The reception section 102 performs various reception processes. For example, the reception section 102 performs a process of receiving operation input by the user. The reception section 102 also performs a process of acquiring motion information and vital information of the user detected by the detection device 600. The process of acquiring motion information and vital information is performed, for example, via the communication section 196.

The game processing section 104 performs various types of game processing for allowing the user to play the game. Examples of the game processes include a process of starting the game when a game start condition is satisfied, a process of making the started game progress, a process of ending the game when a game end condition is satisfied, and a process of calculating a game score. The game processing section 104 performs a game process based on game information such as game data and game program stored in the game information storage section 172.

For example, for a browser game, the game processing section 104 manages user personal information for each user, thereby controlling the progress of the game for each user. The user personal information of the user is stored in the user personal information storage section 176. For example, the game processing section 104 displays a web page that constitutes a website that provides game services on a terminal device in response to a request from the terminal device. Specifically, a web page is displayed through a web browser in the terminal device. When the user selects a hyperlink on the displayed web page, the game processing section 104 transmits new HTML data corresponding to the hyperlink to the terminal device, and the terminal device displays the web page based on the new HTML data. In this way, web pages are sequentially provided to the terminal device by the game processing section 104 in response to the user's operation, thus allowing the game to be advanced based on the user's operation in the terminal device.

The game processing section 104 also performs a process of determining the content of resistance exercise and aerobic exercise for the user to perform based on the user personal information and the like. The details of resistance exercise and aerobic exercise are discussed later. The game processing section 104 also performs a process of determining the game or game effects to be assigned to the selected exercise. The game processing section 104 performs the game process based on the selected game or game effects.

The management section 112 performs, for example, a user authentication process. For example, the management section 112 performs an authentication process of a user who has logged into the system using a terminal device. The authentication process is performed based on, for example, a password input by the user, account information, or the like. The management section 112 performs various charging processes. The management section 112 performs, for example, a process of determining a charge, a process of creating charging data, and a process of saving charging data. The management section 112 also performs various management processes, such as a process of managing various services or a process of managing various types of information.

For example, a user acquires an account by performing predetermined procedures so as to use a service provided by the server system 500 shown in FIG. 1A or the like. By inputting a password associated with the acquired account and logging in, the user can use various services such as playing of network games including social games, services at game websites, online shopping for items or the like, message exchange between users, and addition of friend user. The management section 112 also performs a process of managing the account information of the user, and the like.

The display processing section 120 performs a process of displaying an image on the display section 190. For example, the display processing section 120 generates image generation data (HTML data, etc.) to generate the image. The sound processing section 130 performs a process of outputting sound from the sound output section 192. For example, the sound processing section 130 generates sound generation data for generating the sound (voice, game sound, sound effect).

The operation section 160 allows the user to input various kinds of information such as operation information, and the function thereof can be implemented by an operation button, a direction designating key, an analog stick, a lever, various sensors (an angular speed sensor, an acceleration sensor, or the like), a microphone, a touch panel display, or the like. When a touch panel display is used, a touch panel serving as the operation section 160 and the display section 190 serving as a display are integrally provided.

The storage section 170 serves as a work area for the processing section 100, the communication section 196, and the like, and its function can be implemented by a semiconductor memory, HDD, SSD, an optical disc device, or the like. The storage section 170 includes a game information storage section 172, an exercise information storage section 174, and a user personal information storage section 176.

The game information storage section 172 stores game information such as the game program and game data. The game information in the present embodiment may include information for performing a first game that involves exercise and information for performing a second game that does not involve exercise.

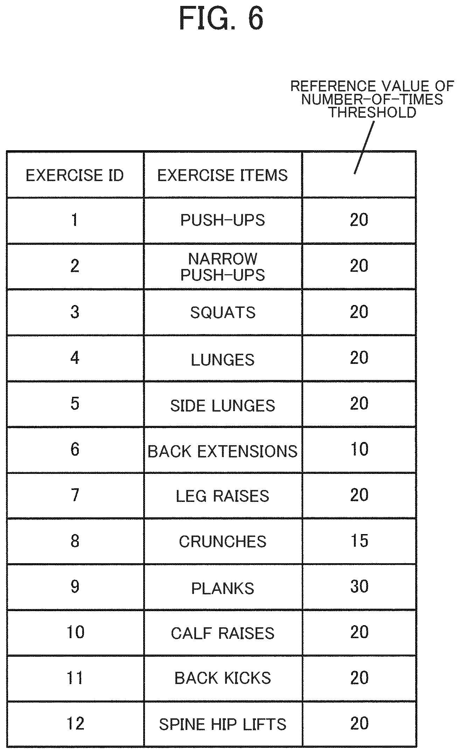

The exercise information storage section 174 stores exercise information, which is information regarding exercise. For example, the exercise information storage section 174 stores, as exercise information, resistance exercise information that defines resistance exercise, aerobic exercise information that defines aerobic exercise, table data for determining an exercise program, and the like. The details of each type of information are described later with reference to FIGS. 6 to 9. However, these information items are not indispensable, and some of them may be omitted. For example, exercise programs may be generated individually from the resistance exercise information and the aerobic exercise information without storing table data in the exercise information storage section 174.

The user personal information storage section 176 stores, as user personal information, personal information (name, gender, date of birth, e-mail address, etc.) of the user. For example, the account information (user ID) of the user is also stored as user personal information. For example, charge information to be processed in the charging process is associated with the account information of each user. The user personal information includes constitutional information indicating anamnesis and the like, as well as physical measurement information such as height and weight. It may also include performance result information that indicates the results of exercise performed by the user.

An information storage medium 180 (computer-readable medium) stores a program, data, and the like. The function of the information storage medium 180 can be implemented by an optical disc, HDD, a semiconductor memory, and the like. The processing section 100 performs various processes of the present embodiment based on programs (data) stored in the information storage medium 180. The information storage medium 180 is capable of storing a program for causing a computer (a device including an operation section, a processing section, a storage section, and an output section) to function as the sections (i.e., a program for causing a computer to execute the processes of the sections) according to the present embodiment.

The display section 190 outputs an image generated according to the present embodiment, and the function thereof can be implemented by an LCD, an organic electroluminescence display, CRT, HMD, or the like.

A sound output section 192 outputs sound generated according to the present embodiment, and the function thereof can be implemented by a speaker, a headphone, or the like.

An interface (I/F) section 194 performs an interface process for a portable information storage medium 195. The function of the I/F section 194 can be implemented by an application-specific integrated circuit (ASIC) or the like for the I/F process. The portable information storage medium 195 is a storage device that stores various types of information from the user, and holds the information without a power supply. The portable information storage medium 195 can be implemented by an integrated circuit (IC) card (memory card), a universal serial bus (USB) memory, a magnetic card, or the like.

The communication section 196 (communication interface) communicates with external apparatuses. The function of the communication section 196 can be implemented by a communication ASIC, hardware such as a communication processor, or communication firmware.

The program (data) for causing the computer to function as the sections according to the present embodiment may be distributed to the information storage medium 180 (or the storage section 170) from an information storage medium of a server system (host device) through a network and the communication section 196. The scope of the present embodiment can include such a configuration where the information storage medium of the server system is used.

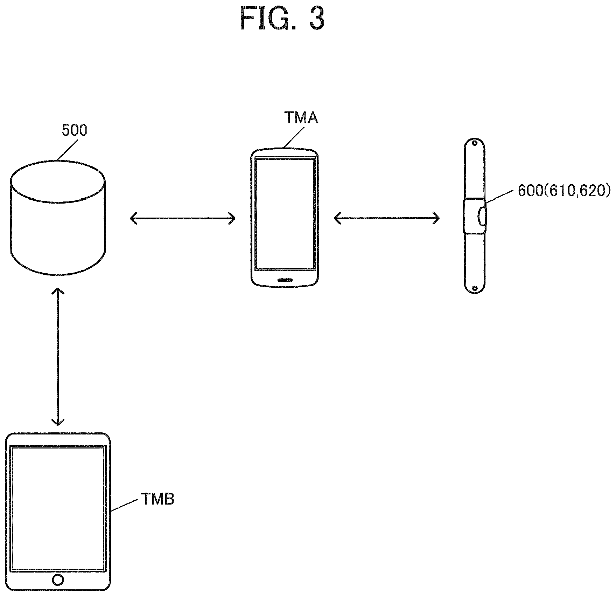

FIG. 3 illustrates a specific example of a system including an exercise management system of the present embodiment. The system shown in FIG. 3 includes a server system 500, a user terminal device TMA, another terminal device TMB, and a detection device 600. The user terminal device TMA corresponds to the terminal device TM described above with reference to FIGS. 1A, 1B, and 2. The server system 500 is similar to the configuration described above. The another terminal device TMB is a terminal device different from the user terminal device TMA. The another terminal device TMB may be, for example, a terminal used by a system administrator of the exercise management system, or a terminal used by medical staff. The medical staff includes a doctor, a nurse, a mentor in exercise therapy, or the like.

The detection device 600 is a device for acquiring detection information that represents the state of the user in exercise. The detection device 600 includes a motion detection section 610 that detects motion information indicating the user's motion, and a vital detection section 620 that detects vital information indicating the user's vital. The motion detection section 610 and the vital detection section 620 may be included in a single detection device 600, or may be included in different detection devices 600. The detection device 600 is a wearable device that can be attached to the user's body with a band, such as the one shown in FIG. 3. The detection device 600 can be attached to the user's wrist, ankle, upper arm, thigh, chest, or any other part of the body. The detection device 600 is not limited to wearable devices. For example, a variation in which the detection device 600 is an image capturing device and detects the motion information of the user based on the captured image, or the like is possible. The user terminal device TMA and the detection device 600 may be provided as a single section. Specifically, the user terminal device TMA may include at least one of the motion detection section 610 and the vital detection section 620.

The motion detection section 610 includes, for example, an acceleration sensor and an angular speed sensor, and outputs, as motion information, information indicating the motion of the part to which the motion detection section 610 is attached. An example in which the output of the motion detection section 610 is information indicating the angle of the attachment part is described below. The angle herein may be, for example, information regarding how much the target part is tilted with respect to a given reference direction. The reference direction is, for example, the vertical direction defined by gravitational acceleration. Alternatively, the reference direction may be the direction of the target part with the user in a given reference posture. The reference posture herein may be an upright posture or a posture at the beginning of the exercise. Since the method of detecting the angle or angle change of the target object based on an acceleration sensor or an angular speed sensor has been known, the detailed explanation thereof is omitted.

The vital detection section 620 acquires vital information of the user. The vital information herein may be any of body temperature, heart rate, blood pressure, respiratory rate, or any other information reflecting the vital activity of the user. Hereinbelow, an example using heart rate (pulse rate) as the vital information is described. The vital detection section 620 includes, for example, a photoelectric sensor with a light-emitting section and a light-receiving section. The light-emitting section irradiates a test object with, for example, green light with a high absorbance of hemoglobin, and the light-receiving section receives reflected light or transmitted light from the test object. The detection result obtained by the light-receiving section is information reflecting the blood flow rate of the test object, and serves as a signal with a periodicity corresponding to the heartbeat interval. Therefore, the heart rate can be estimated based on the output of the photoelectric sensor. Since the method of detecting the heart rate using a photoelectric sensor has been known, the detailed explanation thereof is omitted. The vital detection section 620 may include a sensor of a type different from the photoelectric sensor.

The exercise management system of the present embodiment is, for example, a system to support exercise therapy for patients. For example, a type-2 diabetic patient or a type-2 pre-diabetic patient signs up for the exercise management system based on a referral from a doctor. In registration, the user creates a user account and enters the user personal information such as height, weight, age, and gender. The type-2 pre-diabetic patients may include metabolic syndrome patients (https://www.mayoclinic.org/diseases-conditions/metabolic-syndrome/symptoms-causes/syc-20351916) and borderline metabolic syndrome patients.

The server system 500 instructs the registered user to perform exercises. Specifically, the server system 500 determines an exercise program that includes resistance exercise and aerobic exercise, and transmits information about the exercise program to the user terminal device TMA. The user terminal device TMA presents an exercise program to the user and instructs the user to perform the exercise included in the exercise program. At that time, the user terminal device TMA also instructs the user to wear the detection device 600.

When the user performs an exercise, the detection device 600 detects the motion information and vital information during the exercise and transmits them to the user terminal device TMA. The user terminal device TMA transmits the acquired motion information and vital information to the server system 500. The server system 500 accumulates the motion information and the vital information by associating them with the user account.

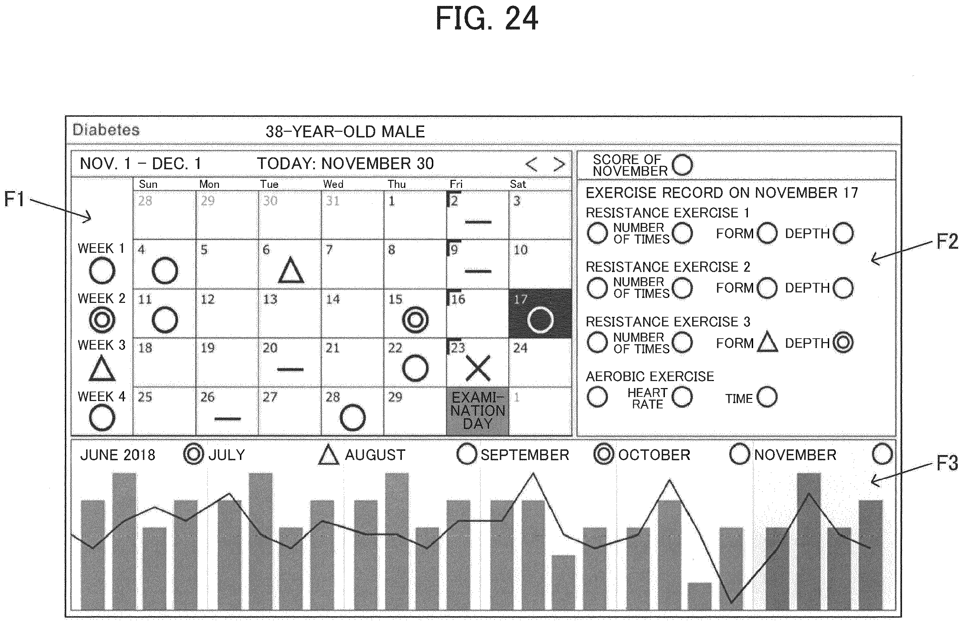

In addition, medical staff such as a doctor acquires performance result information that indicates the results of the exercise performed by the user. Specifically, the server system 500 sends the performance result information of the target user in response to a request from another terminal device TMB. For example, a doctor can use another terminal device TMB to view the performance result information, thereby facilitating the interview with the user (patient). Examples of display screens in another terminal device TMB are described later with reference to FIG. 24.

By using the system shown in FIG. 3, it is possible to provide appropriate support to the user who needs to exercise and to medical staff in charge of the user. In exercise therapy, specialized knowledge is required to set an appropriate exercise content. The exercise content herein means the type and intensity of the exercise (number of times of motions, depth, form, and the like described later). According to the method of the present embodiment, the exercise management system provides instructions regarding the exercise content, thereby enabling the user to perform exercises suitable for exercise therapy more easily. In addition, by using a game process as described below, it is possible to motivate users to exercise and encourage them to perform a continuous exercise.

As shown in FIGS. 1A to 3, the exercise management system of the present embodiment is an exercise management system for managing the exercise of a user who is a type-2 diabetic patient or a type-2 pre-diabetic patient, and includes the storage section 170, the processing section 100, and an acquisition section. The storage section 170 stores user personal information of the user. Specifically, the user personal information is stored in the user personal information storage section 176. The processing section 100 determines the contents of resistance exercise and aerobic exercise based on the user personal information. For example, the game processing section 104 determines an exercise content for each user and carries out a game process based on the determined exercise content. It is also possible that the management section 112 determines an exercise content, and the game processing section 104 performs the game process based on the exercise content.

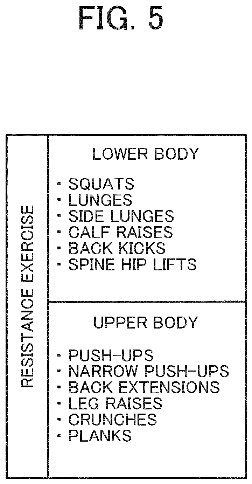

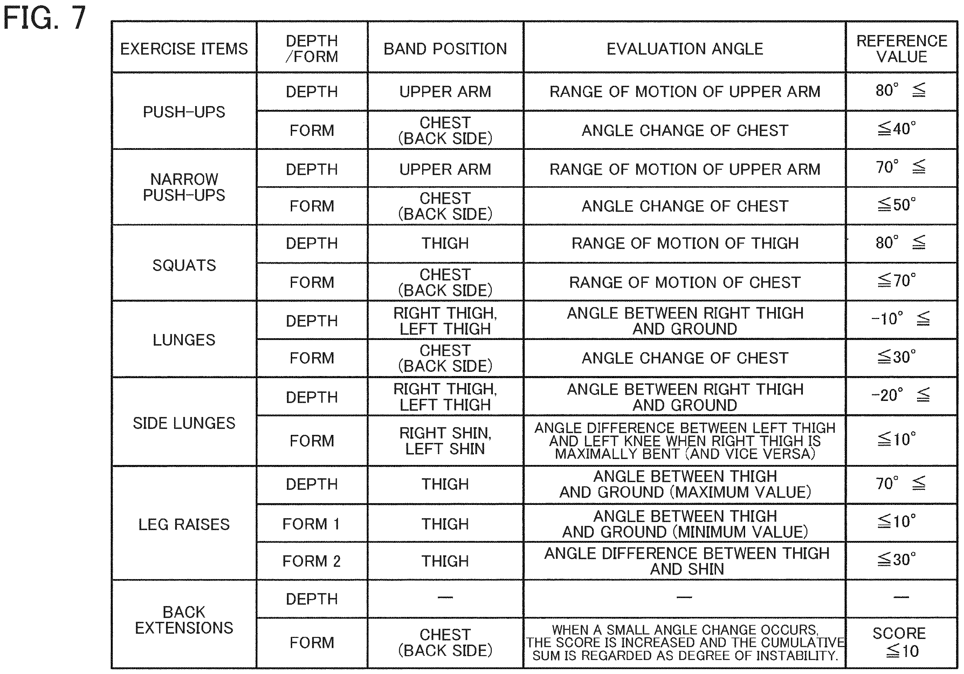

The resistance exercise designates an exercise that applies a load on the muscles (http://exercise.trekeducation.org/resistance-training/what-is-resistance-training/), and includes various types of exercises such as squats, push-ups, and the like, as described later with reference to FIG. 5. In order to make the user properly perform resistance exercise, it is necessary to specify the type of resistance exercise. The resistance exercise content herein designates information that specifies the content of resistance exercise performed by the user, which is, for example, information with which the type of resistance exercise can be specified. For example, in the data described later with reference to FIG. 6, the type of resistance exercise can be uniquely specified by selecting a single exercise ID. The intensity of resistance exercise is also important in order to make the patient perform the resistance exercise in a manner suitable for exercise therapy. The intensity herein requires information regarding the number of times of motions, depth, form, and the like, as described later with reference to FIGS. 6 and 7. In other words, the resistance exercise content may include information of the intensity, in addition to the type of resistance exercise (exercise ID).

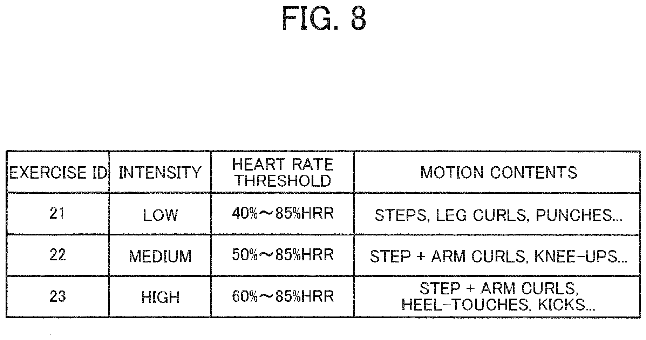

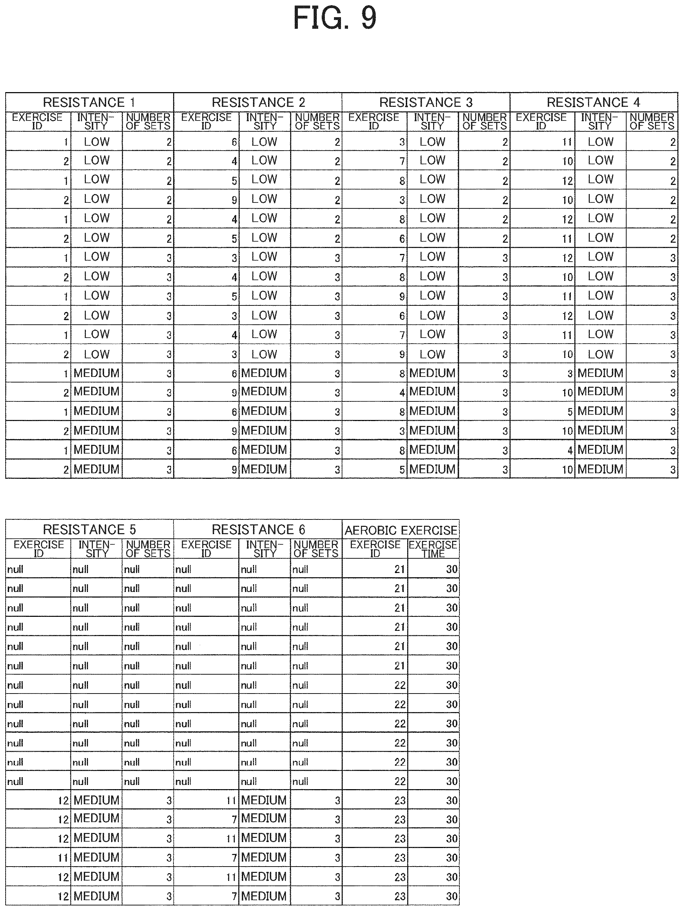

Aerobic exercise refers to muscular movements that require oxygen to burn carbohydrates and fat for energy production (Harvard Health Letter, Glossary of exercise terms, https://www.health.harvard.edu/newsletter_article/Glossary-of-exercise-terms). Aerobic exercise is a relatively light-loaded exercise continued for a long time, such as walking, jogging, aerobics, cycling, and swimming. The aerobic exercise content herein designates information that specifies the content of aerobic exercise performed by the user, which is, for example, information with which the type of aerobic exercise can be specified. For example, the aerobic exercise content includes information for specifying one of walking, jogging, and the like described above. However, as described later with reference to FIG. 8, aerobic exercise may be an exercise that can be easily performed even outside facilities dedicated for exercises, such as aerobics or dance combined with steps, arm curl, and other movements. In this case, the aerobic exercise content includes information specifying the motion content. The aerobic exercise content may also include information regarding intensity. The intensity of aerobic exercise may include information about the duration of the exercise (the exercise time shown in FIG. 9 described later) or heart rate.

The acquisition section acquires motion information indicating the user's motion when the exercise instruction based on the resistance exercise content is given, as well as vital information indicating the user's vital when the exercise instruction based on the aerobic exercise content is given. Specifically, the acquisition section is a communication interface for acquiring information from the detection device 600, and corresponds to the communication section 196. The acquisition section herein may be a communication interface of the user terminal device TMA, or a communication interface of the server system 500. The motion information and the vital information detected by the detection device 600 may be transmitted to the server system 500 via the user terminal device TMA, or may be transmitted directly to the server system 500.

Based on the motion information and the vital information, the processing section 100 of the present embodiment performs a process of presenting the performance result information indicating the performance results of resistance exercise and aerobic exercise by the user on the user terminal device TMA used by the user, or another terminal device TMB.

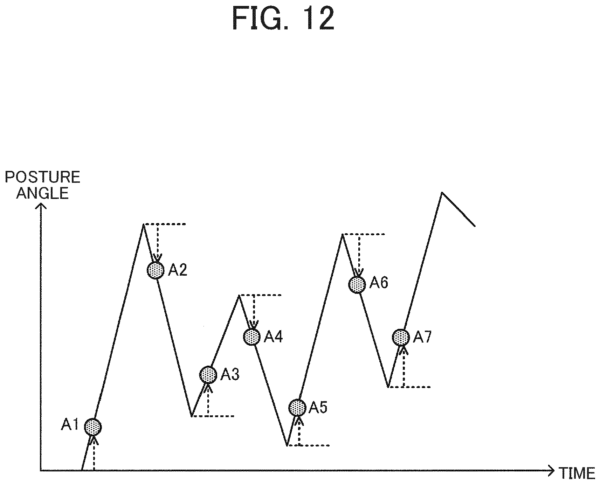

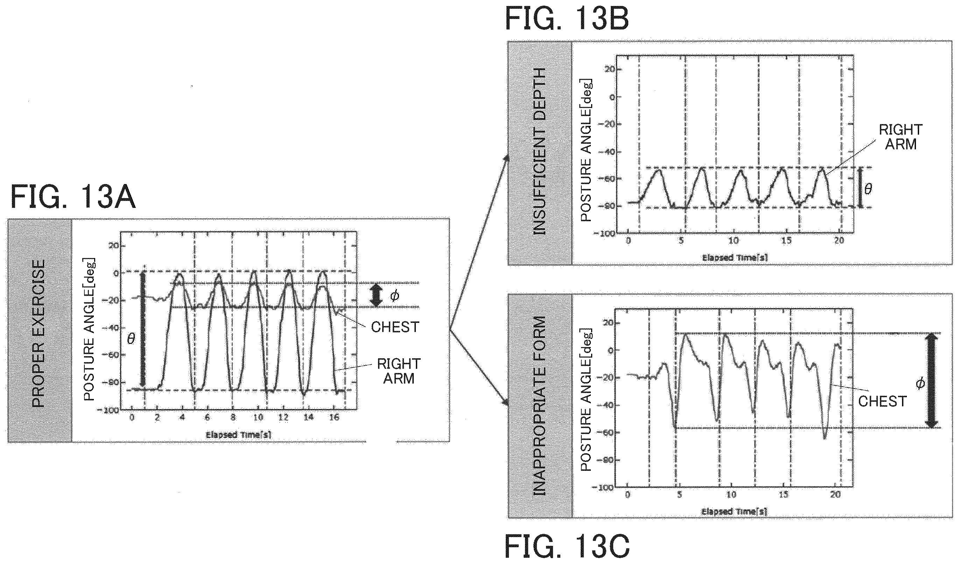

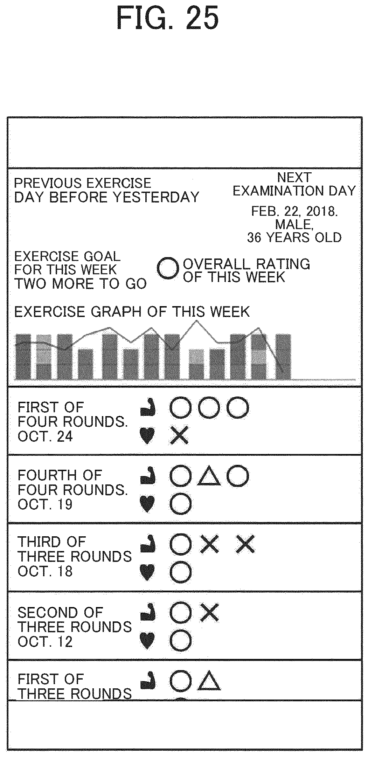

The performance result information herein may be the motion information and the vital information, or may otherwise be information that can be obtained based on the motion information and the vital information. For example, the performance result information may be a graph showing changes of posture angle over time, may be information of the number of times of motions counted by the method described later with reference to FIG. 12, or may be information indicating the results of depth determination and form determination made by the method described later with reference to FIG. 13. Alternatively, the performance result information may be a score, such as ○, △, ×, and the like, which is described later with reference to FIGS. 24 and 25. The performance result information may be information indicating performance results of a single exercise program described later, or may be information indicating performance results for each of the resistance exercise and the aerobic exercise included in a single exercise program. The performance result information may also be information indicating the results of a plurality of exercise programs in chronological order. The performance result information may include various forms of information.

The method of the present embodiment enables a user, who is a type-2 diabetic patient or a type-2 pre-diabetic patient, to perform exercises combining resistance exercise and aerobic exercise suitable for exercise therapy. In this case, the user receives instruction regarding the exercise content. Therefore, even users who do not have specialized knowledge of exercise can easily perform an appropriate content (type and intensity) of exercise. In addition, the exercise performance results can be detected by the motion information and the vital information and can be presented on an appropriate terminal device. By using the user terminal device TMA, it is possible to provide feedback on the performance results to the person who performed the exercise, thereby encouraging him/her to perform a continuous exercise. In addition, by using another terminal device TMB used by medical staff or the like, it is possible to facilitate collaboration between the user, who is a patient, and medical staff.

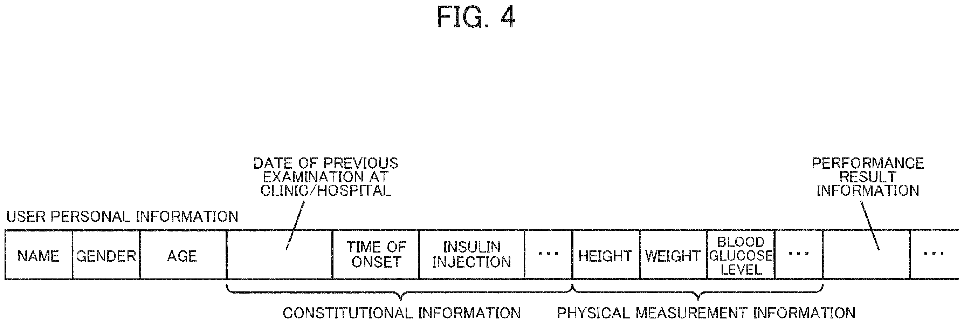

The user personal information of the present embodiment may also include at least one of the user's gender, age, constitutional information, and physical measurement information, as described later with reference to FIG. 4.

In this way, it is possible to present an exercise content that was selected in consideration of individual differences between users. For example, it is possible to prevent the loss of motivation or injuries due to excessively intense exercises.

The user personal information of the present embodiment also includes performance result information of at least one of the resistance exercise and the aerobic exercise performed by the user in the past. The performance result information herein may have various forms, as described above.

In this way, it is possible to present an exercise content that was selected in consideration of specific performance results of the user. For example, by referring to the history of success/failure in exercises, it is possible to improve the user's motivation to exercise by excluding poorly-performed exercises.

The resistance exercise content and the aerobic exercise content to be performed in a single exercise determined by the processing section 100 may be the exercise program described later with reference to FIG. 9. The exercise program consists of a resistance exercise content corresponding to a type of resistance exercise selected from among a plurality of types of resistance exercises, and an aerobic exercise content corresponding to a type of aerobic exercise selected from among a plurality of types of aerobic exercises.

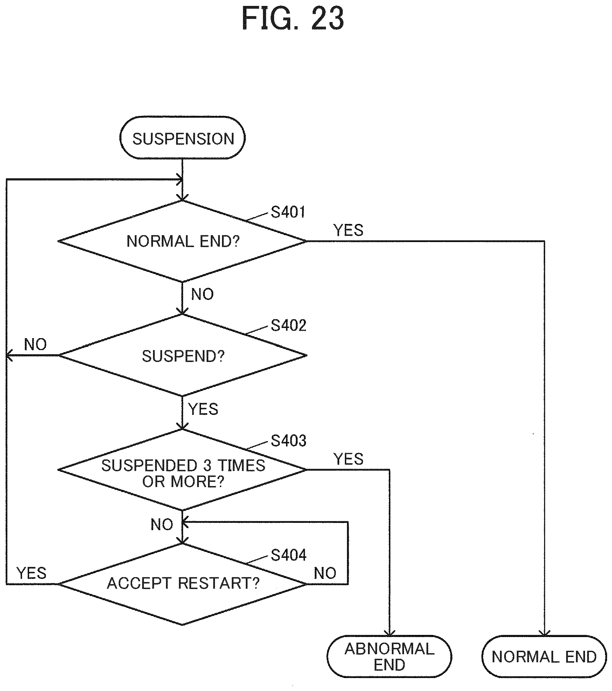

In this way, it is possible to have the user perform a combination of resistance exercise and aerobic exercise in a single exercise. A single exercise refers to an exercise that is performed in a relatively short period of time, or in a narrower sense, an exercise that is performed continuously. However, suspension in the middle of a single exercise is acceptable, as described later with reference to FIG. 23. According to the example described later, a single exercise is completed within up to 48 hours. In this embodiment, the information to make the user perform the single exercise described above is referred to as an exercise program. It is possible to generate a variety of exercise programs by preparing a plurality of types of resistance exercises and a plurality of types of aerobic exercises and setting an exercise content to perform exercises selected from them as an exercise program.

The processing section 100 of the present embodiment may also determine a plurality of types of exercise programs to be performed by the user in a given exercise period based on the user personal information.

In exercise therapy, it is important to continue the exercise for a certain period of time, for example, for several months. By using a plurality of types of exercise programs, it is possible to make the user perform the exercise on a long-term basis. In this case, by providing a variety of exercise programs, it is possible to prevent users from being tired of the exercise and have them perform exercise continuously. It is also possible to generate an exercise program, for example, in consideration of the duration of the exercise, such as increasing the intensity as the exercise continues.

The processing section 100 of the present embodiment may also determine the resistance exercise content and the aerobic exercise content based on input from another terminal device TMB.

In this way, the exercise content can be determined based on input of medical staff or the like. For example, it is possible to modify the exercise program based on expert knowledge. In addition, the another terminal device TMB may be a terminal device used by a system administrator of the exercise management system. In this case, the exercise content can be determined and/or modified according to the updates of data or programs.

When the period or the number of times of the exercise performed by the user is input from the user terminal device TMA or another terminal device TMB, the processing section 100 of the present embodiment may determine the resistance exercise content and the aerobic exercise content based on the exercise period or the number of times of the exercise.

The exercise period herein designates the period for which the user intends to continue the exercise, and indicates a deadline for achieving some results, such as improving the values of physical measurement information. The end of the exercise period is not limited to the end of the exercise therapy, but can also be a timing corresponding to a regular checkup in the middle of the exercise therapy. The number of times of the exercise refers to the number of exercises that the user plans to perform in the period of time in which the user performs continuous exercise. The number of times of the exercise herein is counted for every single exercise described above, and in a narrow sense, it corresponds to the number of exercise programs determined.

In this way, the exercise content can be determined according to the specified exercise period or the number of times of the exercise. For example, if a relatively short exercise period is specified, the exercise content with higher intensity is selected. In this way, it is possible to determine the exercise content capable of expecting a certain result with the specified exercise period or the number of times of the exercise.

The storage section 170 of the present embodiment may also store table data in which a resistance exercise content and an aerobic exercise content are associated with each other, as described later with reference to FIG. 9.

In this way, one set of data in the table data (for example, a single row of data in FIG. 9) represents a single exercise program. Using table data makes the process of determining the exercise program easier. Storing table data, in other words, means storing a plurality of options of exercise programs.

When one of the resistance exercise content or the aerobic exercise content is determined, the processing section 100 of the present embodiment may also determine the other one of the resistance exercise content and aerobic exercise content based on the selected resistance exercise content or aerobic exercise content.

It is also possible to select the resistance exercise content and the aerobic exercise content at the same time, as in the example of selecting a single row from the table data. However, there may be cases with a necessity to give priority to perform a certain type of resistance exercise, or a necessity to perform a certain type of resistance exercise with a specific intensity. In this case, the resistance exercise content is determined first, and then the aerobic exercise content is determined accordingly. This makes it possible to include a resistance exercise content with high priority and an aerobic exercise content with high affinity to the resistance exercise content, in the exercise program. The same applies to the case where the aerobic exercise content is determined first.

The processing section 100 of the present embodiment may also determine the prize to be given to the user based on the performance result information of the user for exercise instructions based on the resistance exercise content and the aerobic exercise content.

In this way, prizes can be given according to the performance result information. For example, if the degree of achievement for the exercise instructions is high, more prize is given compared with the case where the degree of achievement is low. In this way, the acquisition of prizes will serve as motivation, thus it is possible to lead the user to perform the appropriate exercise.

The processing section 100 of the present embodiment may also perform a process of setting friend users, who are other users or virtual users who perform exercises of the resistance exercise content and the aerobic exercise content for which the user is instructed to perform.

The other users herein refer to users other than the target user, and are real users. A virtual user is a non-existent user. For example, the virtual user is a user associated with performance result information generated virtually by the processing section 100.

In this way, it is possible to have the user interact and compete with friend users, thereby improving users' motivation to exercise.

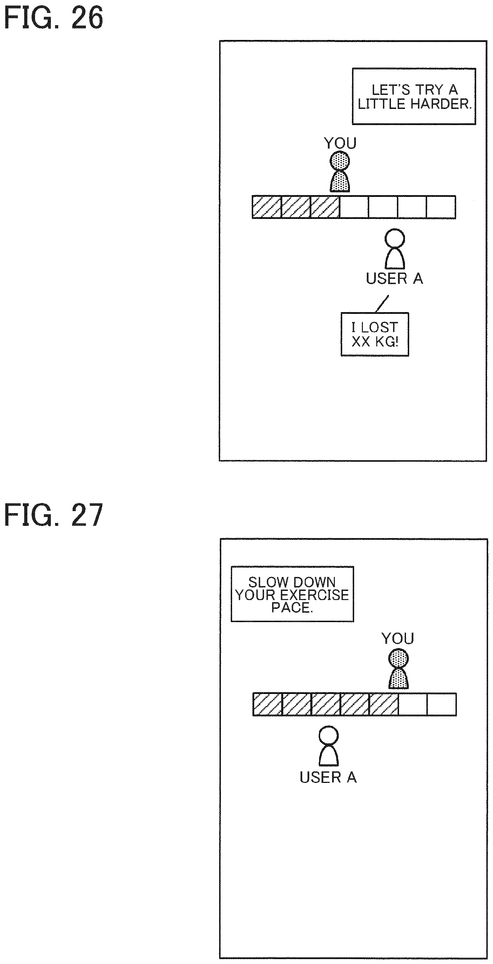

The processing section 100 of the present embodiment may also perform a process of presenting information to encourage the user to exercise based on the difference between the performance result information of the user and the performance result information of a friend user, as shown in FIG. 26. The information to encourage exercise may be only a difference (e.g., the graph in FIG. 26), or may be with additional text or images that directly encourage exercise.

In this way, it is possible to indicate that the user is not performing the exercise with sufficient frequency or intensity by using the difference from a friend user. Therefore, it is possible to increase the effect of motivating the user to exercise, compared to the case where only the performance result information of the user is displayed.

When the degree of achievement for the exercise instructions is determined to be higher than a given standard based on the performance result information of the user, the processing section 100 of the present embodiment may perform a process of skipping the presentation of the information that encourages the user to exercise based on the difference, or may perform a process of presenting information that suggests that the user can reduce the exercise based on the difference as shown in FIG. 27. The degree of achievement with respect to exercise instructions refers to information that indicates whether or not the exercise program instructed to be performed is being performed at an appropriate frequency, or whether or not the exercise program instructed to be performed is being performed at a specified intensity. For example, it is possible to score the performance results according to whether or not the specified intensity was achieved, as described later with reference to FIG. 24. In this embodiment, for example, the degree of achievement is expressed as a score, and it is determined whether or not the degree of achievement is higher than the standard by a comparison between the score and a given threshold value.

As shown in FIG. 26, it is possible to encourage the user to exercise by displaying the difference between the user and a friend user with a higher degree of achievement than the user. However, excessive exercise may damage the user's health contrary to intentions. In the present embodiment, it is possible to prevent the excessive exercise by omitting the display of the difference in the first place or by displaying the difference with a friend user having a lower degree of achievement than the target user.

The processing section 100 of the present embodiment may also present a first performance result information obtained based on the motion information, the vital information and a first determination criteria on the user terminal device, and present a second performance result information obtained based on the motion information, the vital information, and a second determination criteria, which is different from the first determination criteria, on another terminal device TMB.

The processing section 100 of the present embodiment may also perform a process of acquiring reference data indicating standard performance result information of the resistance exercise content and the aerobic exercise content of the exercise that the user has been instructed to perform, and presenting information to encourage the user to exercise based on the difference between the performance result information of the user and the reference data. The processing section 100 may also perform a process of presenting information that suggests that the user can reduce the exercise based on the difference between the performance result information of the user and the reference data.

The reference data, which is standard performance result information, may be, for example, an average value of performance result information of a plurality of users. The reference data may also be information that has been set in advance and accumulated in the storage section 170. The reference data may be information determined according to gender, age, physical measurement information, and the like. In other words, insofar as the reference data is performance result information that can serve as a standard for the target user performing the target exercise program, the method for obtaining the reference data can be changed in various ways. In this way, it is possible to display a difference with respect to desirable performance result, thereby increasing the effect of increasing motivation of the user to perform exercises or the effect of preventing excessive exercise, compared with the case where only the performance result information of the user is displayed.

The first determination criteria and the second determination criteria herein are criteria for determining, based on the motion information and the vital information, whether or not the resistance exercise and the aerobic exercise performed by the user satisfy the given condition. More specifically, the determination criteria specify the intensity. The determination criteria with regard to motion information include a number-of-times threshold, a depth threshold, and a form threshold, and the determination criteria with regard to vital information include a heart rate threshold and exercise time.

In this way, it is possible to present different items of performance result information to the user and to medical staff. For example, by providing relatively easily-achievable first determination criteria, it is possible to give the user a successful experience and encourage him/her to continue the exercise. Further, by using evidence-based criteria as the second determination criteria, it is possible to present medically precise information to medical staff.

The exercise management system of the present embodiment may further include a motion detection section 610 for detecting motion information and a vital detection section 620 for detecting vital information. The motion detection section 610 and the vital detection section 620 may be provided in a single wearable terminal device attached to the user. In other words, the detection device 600 may be a wearable terminal device including the motion detection section 610 and the vital detection section 620.

In this way, it is possible to detect both the motion information and the vital information by wearing a single wearable terminal device, thus easily making the user perform a combination of the resistance exercise and the aerobic exercise.

The method of the present embodiment may also be applied to the server system 500. The server system 500 is a server system for managing exercise of a user, who is a type-2 diabetic patient or a type-2 pre-diabetic patient, and includes a storage section 170 for storing user personal information of the user; a processing section 100 for determining, based on the user personal information, the resistance exercise content, which is information to specify the content of the resistance exercise performed by the user, and the aerobic exercise content, which is information to specify the content of the aerobic exercise performed by the user; and an acquisition section for acquiring motion information indicating the motion of the user when the exercise instructions are given based on the resistance exercise content, and vital information indicating the user's vitals when the exercise instructions are given based on the aerobic exercise content. Based on the motion information and the vital information, the processing section 100 performs a process of presenting the performance result information indicating the performance results of resistance exercise and aerobic exercise by the user on the user terminal device TMA used by the user, or another terminal device TMB.

The method of the present embodiment may also be applied to the terminal device TM (user terminal device TMA). The terminal device TM is a terminal device used for exercise management of a user, who is a type-2 diabetic patient or a type-2 pre-diabetic patient, and includes a processing section 100 that performs a first acquisition process to acquire a resistance exercise content and an aerobic exercise content determined based on the user personal information of the user, an instruction process to provide exercise instructions based on the resistance exercise content and the aerobic exercise content to the user, and a second acquisition process to acquire motion information indicating the motion of the user when the exercise instructions are given based on the resistance exercise content and vital information indicating the user's vitals when the exercise instructions are given based on the aerobic exercise content; and a presentation section for presenting performance result information indicating the performance results of the resistance exercise and the aerobic exercise performed by the user based on the motion information and the vital information. Specifically, the presentation section may be the display section 190 or the sound output section 192, or may be both of them. The first acquisition process herein refers to, in a narrow sense, a process of controlling the communication interface that communicates with the server system 500, and the second acquisition process refers to a process of controlling the communication interface that communicates with the detection device 600. The two communication interfaces used herein can be one or two interfaces that perform communication according to the same communication standard, or two interfaces that perform communication according to different communication standards.

The terminal device TM described above may also perform a game process. The game processes in the terminal device TM include a process of transmitting operation information indicating an operation input by a user to the server system 500, and a process of receiving information used for output in the display section 190 and the sound output section 192 from the server system 500. For example, as in a browser game, the terminal device TM may function as an input/output interface with the user, and the specific determination process regarding the start, the progress, and the end of the game may be performed by the server system 500. The game processes of the present embodiment include processes in the terminal device TM in an embodiment similar to the present embodiment.

2. Method According to the Present Embodiment

Next, a method according to the present embodiment is described in detail. The exercise management system of the present embodiment stores the user personal information, performs a process of determining the resistance exercise content and the aerobic exercise content based on the user personal information, acquires the motion information and the vital information when the exercise instructions are given, and presents the performance result information on the user terminal device TMA or another terminal device TMB based on the motion information and the vital information.

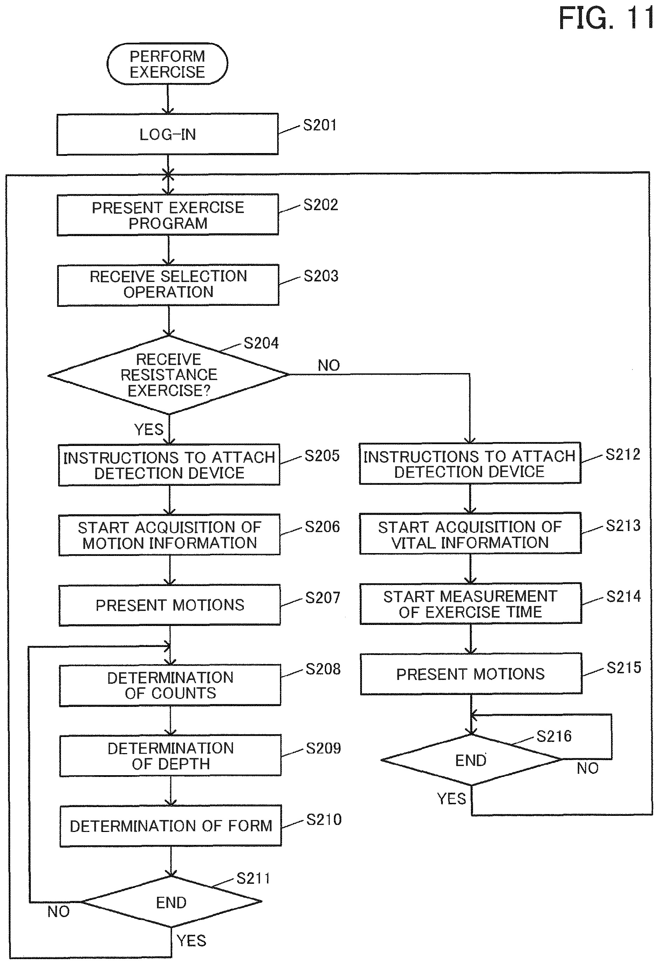

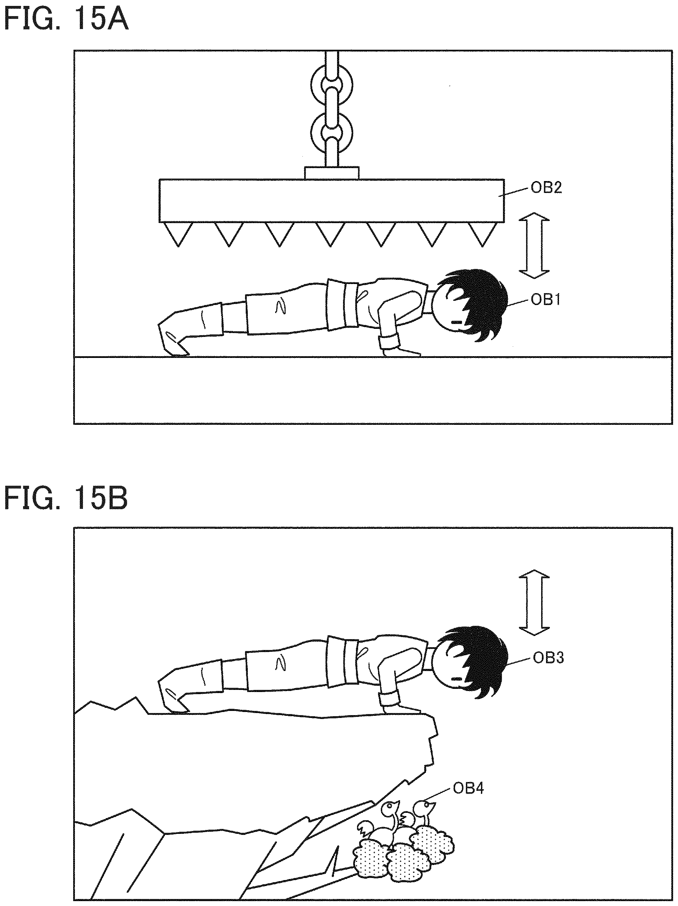

The method of the present embodiment makes it possible to instruct a user to perform exercises combining resistance exercise and aerobic exercise. In addition, since the user personal information is used to determine the exercise content, it is possible to provide exercise instructions reflecting individual differences of the users. The user personal information is described later with reference to FIG. 4. The process of determining the exercise content is described later with reference to FIG. 9. The performance result information is described later with reference to FIGS. 11 to 14. The presentation process in the terminal device is described later with reference to FIGS. 24 and 25. The presentation on the user terminal device TMA may be display of a game screen, as described later with reference to FIGS. 15A to 16B and 20.

In addition, the exercise management system of the present embodiment is capable of performing game processes for executing the first game and the second game. The exercise management system obtains detection information when the user performs exercises in the first game, performs a game process for executing the first game based on the performance result information, sets a given rest period after the execution of the first game, and does not allow the execution of the first game and allows the execution of the second game during the rest period.

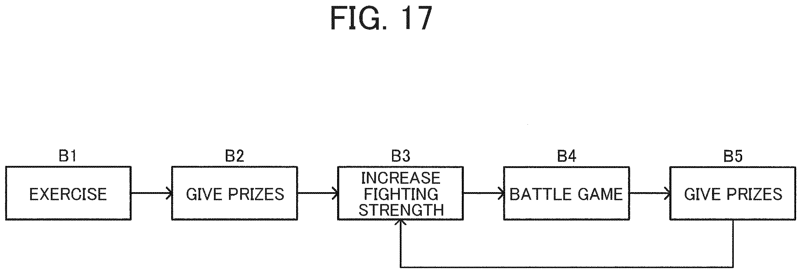

With the method of the present embodiment providing a rest period, the user can be prevented from performing the excessive exercise. It is also possible to maintain the user's interest in the game by allowing a second game during the rest period. The details are described later with reference to FIGS. 17 to 21.

Further, in the present embodiment, a plurality of games or a plurality of game effects are associated with each of the plurality of types of exercises. The exercise management system of the present embodiment selects a game or game effects to be used for the game process from among a plurality of games or game effects associated with exercises. Then, the detection information is acquired during the selected game or game effects, and a game process using the selected game or game effects is executed (the game is advanced) based on the performance result information.

According to the method of the present embodiment, a plurality of games or a plurality of game effects are associated with a single type of exercise. Therefore, the user's interest in the game can be maintained even when the same type of exercise is performed a plurality of times. The details are described later with reference to FIGS. 15A to 16B.

2.1 User Personal Information

FIG. 4 illustrates an example of a data structure of the user personal information. As shown in FIG. 4, the user personal information includes the user's name, gender, age, constitutional information, and physical measurement information. Constitutional information indicates anamnesis and/or medication history. For example, constitutional information includes the date of the previous examination at clinic/hospital, the time of onset of diabetes, whether or not the patient is taking insulin injections, current medications, and the like. Physical measurement information is obtained by the measurement of the user. For example, physical measurement information includes height, weight, blood glucose level, and the like. The physical measurement information may also include HbA1C, blood pressure, waist size, triglyceride level, cholesterol level, and other types of information not shown in the diagram.

The user personal information also includes performance result information that indicates the results of exercise performed by the user. The performance result information includes various forms of information based on the motion information and the vital information, as described above. In this way, the performance results for each user can be appropriately managed.

In the present embodiment, the exercise management system determines the exercise content and gives instructions to perform the exercise, thereby encouraging the user to perform an appropriate exercise. Appropriate exercise depends on the user's physical strength, lifestyle, the progress of diabetes, and the like. Therefore, the exercise content that the user is instructed to perform may be managed for each user, and information indicating the exercise content may be included in the user personal information. For example, the user personal information may include one or more exercise programs associated with the target user. Specifically, the performance result information shown in FIG. 4 may be stored by being associated with an exercise program. Further, an exercise program generated for instructions for future exercise may also be managed as the user personal information. The details of the exercise program are described later.

2.2 Resistance Exercise

FIG. 5 illustrates an example of resistance exercise according to the present embodiment. Resistance exercise repeatedly or continuously stresses the muscles. As shown in FIG. 5, squats, lunges, side lunges, calf raises, back kicks, and spine hip lifts are known as resistance exercises that stress the muscles of the lower body. Also, push-ups, narrow push-ups, back extensions, leg raises, crunches, and planks are known as resistance exercises that stress the muscles of the upper body. The resistance exercise can be thus classified by the upper body and the lower body, and can also be classified according to the size of the muscle to be stressed (large muscle group, small muscle group). Since the resistance exercise has been known, a detailed description thereof is omitted. In addition, although FIG. 5 illustrates 12 types of resistance exercises, some resistance exercises may be omitted and other resistance exercises may be added.

The exercise management system of the present embodiment stores, for example, information regarding the 12 types of resistance exercise described above. Then, the exercise management system instructs the user to perform the resistance exercise selected from among the 12 types of resistance exercise.

For example, the exercise information storage section 174 stores the resistance exercise information shown in FIGS. 6 and 7 as exercise information. Note that FIG. 7 shows examples of information regarding some types of resistance exercise. The resistance exercise information includes the exercise ID, exercise items, the band position, the reference value for number-of-times threshold, the reference value for depth threshold, and the reference value for form threshold. Although FIGS. 6 and 7 are referred to as examples of resistance exercise information, the database configuration is not limited to these examples. In other words, the specific data structure of the resistance exercise information can be changed in various ways. For example, it is also possible to arrange such that the information of evaluation angle is not stored in the exercise information storage section 174, and is stored in the game information storage section 172 as a part of the depth determination program or form determination program.

The exercise ID is information for uniquely identifying the resistance exercise. The exercise item is the name of the resistance exercise, and is, for example, a character string of "squats" or the like described above. The band position is information indicating the attachment position of the detection device 600 when the target resistance exercise is performed. The band position is information for specifying a site to which the detection device 600 is attached, such as the upper arm, chest (back), thigh, shin or the like. In this example, two detection devices 600 are used to enable determination of both depth and form as described later. However, the user may wear only one of the detection devices 600.

The number of times of motions, the depth, and the form of the resistance exercise are described below. In many of the resistance exercises, the action of moving from a start posture to a different posture and then returning to the start posture is repeated. For example, for push-ups, the start posture is a prone position with the arms extended in which the body is supported by the palms and the toes (or knees). After the start posture, the body is sunk by bending the elbows, and then the body is returned to the start posture by again extending the elbows. This sequence of movements is counted as one motion. The number-of-times threshold is a threshold indicating the number of times of the resistance exercise that need to be done to determine that the resistance exercise is completed. For example, when the number-of-times threshold of push-up is 20, a single set of push-ups is determined to be completed on the condition that bending and extending of the elbows by the user are detected 20 times. The details of the process of counting the number of times of motions are described later with reference to FIG. 12.

When the number of times of motions is too small, the load applied to the muscles is insufficient, and is not desirable for exercise therapy. More specifically, in the resistance exercise, it is possible to set a number of times of motions desired for exercise therapy. The reference value of the number-of-times threshold in the present embodiment is information indicating an evidence-based number of times of motions appropriate for exercise therapy. By carrying out determination using the number-of-times threshold during the exercise of the user, it is possible to have the user perform an effective resistance exercise.

The reference value of the number-of-times threshold described above may be used as a fixed number-of-times threshold to be used when the user is instructed to perform the resistance exercise. However, conditions such as age, gender, exercise habits, and degrees of progress of diabetes vary between the users. Further, even the same user may experience a decrease in weight or an increase in muscle strength or endurance due to continuous exercise, and he/she may become capable of exercises with higher intensity. Therefore, when instructing the user to perform resistance exercise, the number-of-times threshold may be varied depending on the situation. For example, the reference value is the lowest value of the number of times of motions for which evidenced efficacy for exercise therapy is ensured, and a value larger than the reference value may be set as the actual number-of-times threshold. In this case, the exercise information storage section 174 may include information that is not shown in FIGS. 6 and 7, such as a condition for changing the number-of-times threshold, a variation range for each time, the upper limit value of the number-of-times threshold that can be set, and the like. However, the reference value may be set as a numerical range, and information for setting the number-of-times threshold may be provided in various forms.

A single instruction to perform the resistance exercise can also give instructions to perform a plurality of sets of resistance exercises. For example, possible embodiments include an instruction to perform two sets of push-ups wherein each set has 20 push-ups. The interval between the sets is, for example, a short period of time, i.e., about several tens of seconds. An example in which the number of sets is managed as information different from the number-of-times threshold is described below. However, the number-of-times threshold may be information including the number of times of motions for each set and the number of sets.

In addition, in the resistance exercise, the magnitude of the load changes in accordance with the actual movement of the user. For example, in push-ups, the elbow is extended in the start posture, and the muscle is loaded by bending the elbow. Therefore, bending the elbow to a certain angle is a condition for applying an appropriate load.

The depth threshold in the present embodiment is a threshold for determining whether or not the main movable parts are moving to a certain extent to apply sufficient load during the target resistance exercise. In the example of push-ups described above, the range of motion (angle variation amount) of the upper arm is detected as an evaluation angle by using the detection device 600 attached to the upper arm. Then, a value close to 90 °, for example, 80 °, is set as the depth threshold, and it is determined whether or not the evaluation angle is equal to or greater than the depth threshold. When the evaluation angle exceeds the depth threshold, it is determined that a single push-up is performed with an appropriate depth. In the example of push-ups, the depth is determined to be appropriate when the evaluation angle is equal to or greater than the depth threshold. However, depending on the setting of the evaluation angle or the depth threshold, the depth may be determined to be appropriate when the evaluation angle is equal to or smaller than the depth threshold. The depth threshold may include a lower-limit depth threshold and an upper-limit depth threshold, and the depth may be determined to be appropriate when the evaluation angle is equal to or greater than the lower-limit depth threshold and equal to or smaller than the upper-limit depth threshold.

The reference value of the depth threshold in the push-ups is, for example, 80 ° described above. The reference value of the depth threshold described above may be used as a fixed depth threshold to be used when the user is instructed to perform the resistance exercise. However, similarly to the example of the number-of-times threshold described above, the depth threshold may be made variable in consideration of individual differences or the like of each user, and the exercise information storage section 174 may store information for making the depth threshold variable (not shown).

In addition, in some resistance exercises, a motion other than the main movable parts may affect the load. For example, in the case of push-ups, it is necessary to keep the body straight even during the motion of bending and extending the elbows. Specifically, it is desirable to maintain a posture in which the back, the waist, the hip, and the toes (or the knees when the knees touch the floor) are linearly aligned. It is not possible to apply a sufficient load to the muscles when the user has a posture with the waist raising or the back deflected. That is, in order to make the user perform appropriate resistance exercise, it is desirable to also determine motions other than the motion related to the depth determination.

The form threshold in the present embodiment is a threshold for determining whether or not motions other than the motion (posture, angle) related to the depth determination are appropriate. In a narrow sense, in the case where the determination using the depth threshold is performed by the detection device 600 attached to a first site, determination using the form threshold is performed by the detection device 600 attached to a second site, which is different from the first site. However, as in leg raises described later, both the depth determination and the form determination may be performed by the detection device 600 attached to a given site. In the example of leg raises, the angle of the thigh in a first posture is used for the depth determination, and the angle of the thigh in a second posture is used for the form determination.

For example, the form threshold value of push-ups is an upper limit of the variation of the angle of the chest. As described above, when the user is keeping a linear posture, since the movement of the chest is limited to the movement following the bending and extending of the elbows, the angle change is small. On the other hand, when the waist is raised, the variation of the angle of the chest is large. Therefore, the variation of the angle of the chest is determined to be used as an evaluation angle. When the evaluation angle is equal to or smaller than the form threshold, it is determined that a single push-up is performed with an appropriate form.

The reference value of the form threshold in push-ups is, for example, 40°. The reference value of the form threshold described above may be used as a fixed form threshold to be used when the user is instructed to perform the resistance exercise. However, similarly to the examples of the number-of-times threshold and the depth threshold described above, the form threshold may be made variable in consideration of individual difference or the like of each user, and the exercise information storage section 174 may store information for making the form threshold variable (not shown).

In addition, there is the resistance exercise in which a predetermined posture is kept for a certain period of time, such as planks. In this case, the number-of-times threshold is replaced with information indicating a period of time during which the posture must be kept. In addition, in the case of planks, it is not necessary to consider the depth, and the determination is made only with respect to the form. Further, the determination of the form may be omitted depending on the type of the resistance exercise. The depth determination or the form determination may also be performed based on a score calculated based on a given angle, as in the determination regarding the form of back extensions.

In the determination of whether or not the leg raises are appropriately performed, three conditions can be used: the angle between the floor and the thigh with the legs raised is equal to or greater than a predetermined threshold value; the angle of the thigh with the legs lowered is equal to or smaller than a predetermined threshold value; and the knees are extended. In this case, for example, the threshold value related to the angle of the thigh with the legs raised is set as the depth threshold, the threshold value related to the angle of the thigh with the legs lowered is set as a first form threshold, and the threshold value related to the angle of the knee is set as a second form threshold. In this way, a plurality of form thresholds may be used in the form determination.

The intensity of the resistance exercise is not limited to the number of times of motions, the depth, and the form. For example, the intensity may be a performance time that is a time from when the posture is changed from the start posture to when the posture is returned to the start posture. For example, the following assumes a resistance exercise in which the start posture applies relatively a small load to the muscles, and the load on the muscles increases as the posture changes. When the performance time is short and the posture is quickly changed, the state of stressing the muscles ends in a short time, whereas when the performance time is long, the duration of stressing the muscles increases. Therefore, the performance time is also usable as the intensity of the resistance exercise. The performance time is a period starting from a performance detection timing and ending at a timing at which the angle returns to the same angle after counting-up, for example, in the process of counting the number of times of motions (described later with reference to FIG. 12). The performance time may also be, for example, a period from the performance detection timing to the next performance detection timing (for example, between A1 and A3).

2.3 Aerobic Exercise

The exercise information storage section 174 also stores the aerobic exercise information shown in FIG. 8 as exercise information. The aerobic exercise information includes exercise ID, intensity, heart rate threshold, and motion contents. Note that the database configuration is not limited to that in FIG. 8, and various modifications can be made to the specific data structure of the aerobic exercise information.

The exercise ID is information for uniquely identifying the aerobic exercise. The intensity is information indicating the degree of the load applied by the exercise, and is expressed as, for example, three stages of low, medium, and high. The motion contents are information specifying the specific motions of the user during aerobic exercise. The heart rate threshold is a threshold for determining whether or not the target aerobic exercise has been properly performed, and correlates to the intensity.

Motion contents in aerobic exercise are described below. Jogging, running, swimming, and the like have previously been known as aerobic exercises, and the motion content in FIG. 8 may be information specifying these aerobic exercises. However, since the method of the present embodiment assumes exercise therapy for type-2 diabetic patients, and the like, it is necessary to also target users who do not have exercise habits and are not highly motivated to the exercise. Therefore, forcing the user to move to a place suitable for exercise, such as a running course or a gym, can be a factor that inhibits the user from performing the exercise.

Therefore, it is preferable that the motions specified by the motion contents of the present embodiment be easy to perform. More specifically, it is preferable that the motions specified by the motion contents of the present embodiment be performable at home without a large space or special equipment. Examples include thigh-ups, rope jumping, hula hoop, dancing, aerobics, boxercise, and the like. For rope jumping, hula hoop, and boxercise, it is easier to perform these exercises by performing them without using tools such as ropes or mitts. Aerobic exercises are preferably continued for a certain length of time, such as 30 minutes. Therefore, if the user performs a simple aerobic exercise such as only thigh-ups or only rope jumping for 30 minutes, the user is likely to feel bored because there is little variation in the movement. Moreover, the users according to the present embodiment are likely to have little experience in dancing or aerobics; therefore, if they are simply instructed to perform a dance, they may not know what specific movements should be made.

Accordingly, the aerobic exercise of the present embodiment is preferably an exercise in which a plurality of types of motions, such as steps, arm curls, leg curls, and punches are performed in chronological order. Therefore, the aerobic exercise information includes, as the motion contents, the contents regarding the combination of these types of motions, more specifically, the order, the duration, and other types of information of each motion. When instructing the user to perform aerobic exercise, by explaining each motion to the user using images and sounds, even users who are not familiar with exercises can easily perform aerobic exercises.

The heart rate threshold in aerobic exercise is described below. It is known that, in aerobic exercise, fat burning effects and the like are enhanced by exercising while ensuring that the heart rate satisfies a given condition. For example, when the resting heart rate is HRmin, the maximum heart rate is HRmax, and the current heart rate is HR, the ratio information, HRR, is obtained using the following equation (1). The resting heart rate HRmin is the heart rate obtained at rest. For example, the exercise management system performs a presentation process in which the user is instructed to sit on a chair or lie down on a bed at the first time of use, and, while the presentation process is being performed, obtains the resting heart rate HRmin based on the information obtained by the detection device 600. The maximum heart rate HRMax can be determined based on age, for example.

[Math. 1]

$$HRR = \frac{HR - HRmin}{HRmax - HRmin} \times 100$$
$$\cdots \ (1)$$

The heart rate threshold is determined, for example, based on HRR. In the example in FIG. 8, when the intensity is low, the heart rate threshold is set to 40%, which is the target value of HRR. Similarly, when the intensity is medium, HRR = 50% is the heart rate threshold, and when the intensity is high, HRR = 60% is the heart rate threshold. It is also known that, when performing a heavy-load exercise where the heart rate is very high, carbohydrates are more likely to be burned instead of fats. Therefore, the heart rate threshold may include not only a lower limit but also an upper limit. The upper limit of the heart rate threshold is, for example, 85%. By thus setting the heart rate threshold, it is possible to have the user perform an aerobic exercise with an appropriate load. The determination using the heart rate threshold is specifically described later.

Although resistance exercise and aerobic exercise have been described above, warm-up before the exercise and cool-down after the exercise may be performed in at least one of the resistance exercise and the aerobic exercise. In this case, the exercise information storage section 174 stores information about warm-up and cool-down. The contents of the warm-up and cool-down may differ between the resistance exercise and the aerobic exercises.

### 2.4 Exercise Program

In the method of the present embodiment, the resistance exercise content and the aerobic exercise content to be performed in a single exercise may be an exercise program. The exercise program is determined by selecting one or more types of exercise from a plurality of types of resistance exercises and a plurality of types of aerobic exercises, respectively. In this case, table data may be used. Further, one of the resistance exercise content and the aerobic exercise content may be determined first, and then the other may be determined accordingly. The exercise program is also determined based on the user personal information, as described above. However, the exercise program may be determined based on input from another terminal device TMB, exercise period, or the number of times of the exercise. It is also possible to determine a plurality of types of exercise programs to be performed by the user in a given exercise period.

An overview of the exercise program is given below, followed by specific description of the process of determining the exercise program.

### 2.4.1 Overview of Exercise Program

The exercise program is described below. It is known that, in exercise therapy, it is effective to perform both resistance exercise and aerobic exercise in combination, rather than performing only one of them. Therefore, the exercise management system provides the user with instructions to perform a combined exercise of resistance exercise and aerobic exercise. The combination of resistance exercise and aerobic exercise is hereafter referred to as an exercise program.

As described above with reference to FIGS. 5 to 7, the exercise management system of the present embodiment stores information regarding a plurality of types of resistance exercises. The exercise management system generates an exercise program by performing a process of selecting one of the above plurality of types of resistance exercises. More specifically, the selection of the type is the selection of the exercise ID.

The exercise management system also performs a process of determining the intensity of the selected resistance exercise. More specifically, the intensity is the number-of-times threshold, the depth threshold, the form threshold. If the reference value of the number-of-times threshold is a given fixed value, the value is used as the number-of-times threshold, and if the number-of-times threshold is variable within a range of given lower and upper limits, a process of using one of the values within the range as the number-of-times threshold is performed. The same applies to the depth threshold and the form threshold, and a specific value is determined based on the reference value. In the narrow sense, the depth threshold and the form threshold are values representing an angle, and may be an integer or a fractional value.

The intensity of the resistance exercise may be adjusted in predetermined stages, such as low, medium, and high. In this case, the number-of-times threshold, the depth threshold, and the form threshold of each resistance exercise are individually adjustable in up to three stages.

By selecting the type and the intensity of the resistance exercise, a specific exercise content that the user is instructed to perform is determined. The information regarding the selected resistance exercise is hereinafter referred to as a resistance exercise content. The resistance exercise content may include other information associated with the exercise ID, such as information of the band position.

Similarly, the exercise management system generates an exercise program by performing a process of selecting one of the above plurality of types of aerobic exercises. The information regarding the selected aerobic exercise is hereinafter referred to as an aerobic exercise content. In the example described above with reference to FIG. 8, an exercise ID of the aerobic exercise corresponds to an intensity as one-to-one. However, also for aerobic exercise, it is possible to individually select the type and the intensity.

Considering the effects of exercise therapy, such as constitutional improvement, it is desirable to use a set of a plurality of types of resistance exercises and one aerobic exercise as a single unit of exercise. For example, a single exercise program may include four to six kinds of resistance exercise and one kind of aerobic exercise. In this case, the resistance exercise content is information specifying the four to six types of resistance exercise selected, as well as information specifying the intensity or the like of each resistance exercise. In addition, the exercise program may include information specifying the order of performing the four to six kinds of resistance exercise and the aerobic exercise.

The exercise information storage section 174 may store options of exercise programs as exercise information. FIG. 9 illustrates an example of table data representing options of exercise program. As shown in FIG. 9, the table data includes the resistance exercise content and the aerobic exercise content. The resistance exercise content is a combination of four or six items of data. Each of the data items contains an exercise ID, an intensity (low, medium, or high), and the number of sets associated with each other. The aerobic exercise content is data in which exercise ID is associated with exercise time. The exercise time is information representing the duration of a single aerobic exercise.

As shown in FIG. 9, by storing table data in which resistance exercise contents and an aerobic exercise content are associated with each other, it is possible to easily generate exercise programs. Specifically, a single exercise program can be generated by selecting a single row of the table data in FIG. 9. Although FIG. 9 illustrates table data with 18 rows, the number of options of exercise programs included in the table data is not limited to this example.

A single exercise program may have any combination of the plurality of types of resistance exercises. However, the type of resistance exercise may be determined to satisfy given conditions. For example, the table data in FIG. 9 is created so that the data in each row satisfies the given conditions.

Various specific conditions can be assumed. For example, as described above with reference to FIG. 5, the part of the muscles to which loads are applied (upper body, lower body) differs depending on the type of resistance exercise. Considering well-balanced application of loads to all muscles in the body in a single exercise, the resistance exercise content is determined so that the number of resistance exercises that apply loads to the upper body is equal to the number of resistance exercises that apply loads to the lower body. In the case where the order of performing the resistance exercises is determined, for example, the resistance exercises may be determined so that the exercises that load the upper body and those that load the lower body are performed alternately. However, it is also possible to ensure the well-balanced resistance exercise by performing an exercise program that focuses on the upper body and an exercise program that focuses on the lower body alternately. Therefore, only a plurality of resistance exercises that load the upper body may be included in a single exercise program, or only a plurality of resistance exercises that load the lower body may be included in a single exercise program. Further, the types of the plurality of resistance exercises included in a single exercise program may be selected according to the muscle size, considering the well-balanced application of loads to the large muscle groups and the small muscle groups.

Further, as shown in FIG. 6, to determine the number of times of motions, the depth, and the form of the resistance exercise, it is important to attach the detection device 600 to an appropriate part of the user. Therefore, depending on the order of performing the resistance exercise, it may be necessary to re-attach the detection device 600 before the exercise starts. If the position of the detection device 600 needs to be re-attached frequently, the user may feel it troublesome and may lose motivation to exercise. Therefore, the plurality of types of resistance exercises included in a single exercise program may be selected so as to satisfy the condition that the re-attachment of the detection device 600 is less than a predetermined number of times.

In a single exercise, the user performs the resistance exercise and the aerobic exercise specified by a single exercise program. For example, performing exercise about three times a week for several months according to an exercise program is expected to result in effects of, for example, lowering body weight and blood sugar levels. The exercise management system may generate several months' worth of exercise programs (e.g., 36 exercise programs in 12 weeks) in advance, or may generate the next exercise program when one exercise program is completed.

Note that storing the options of exercise programs in the form of table data in advance is not indispensable. For example, the processing section 100 may dynamically generate an exercise program based on the resistance exercise information and the aerobic exercise information shown in FIGS. 5 to 8.

2.4.2 Determination of Exercise Program using User Personal Information

If the table data shown in FIG. 9 is used, the exercise program can be determined by selecting one of the rows, as described above. It is preferable to use the user personal information in this determination.

For example, young users, users with appropriate BMI, and users with exercise habits have a high probability of being capable of performing resistance exercises with a high load. Therefore, it is possible to have such users perform a type of resistance exercise accompanied by intense movement, or it is possible to increase the intensity of the same resistance exercise. On the other hand, if an elderly, obese, or inactive user is instructed to perform high-load resistance exercise, they are not likely to achieve the instructions given, which may result in loss of motivation. For this reason, the resistance exercise content needs to be adjusted according to the user. However, as described above with reference to FIGS. 5 through 7, there are various types of resistance exercises, and there is a wide range of intensity suitable for exercise therapy. Therefore, it is not easy for users who do not have specialized knowledge to determine an appropriate resistance exercise content. The same applies to aerobic exercise. It is preferable to consider the individual differences of the users.

Therefore, the processing section 100 of the exercise management system determines the exercise program based on the user personal information of the target user. As described above with reference to FIG. 4, the user personal information is, for example, the gender and the age of the user. In this way, it becomes possible to create exercise programs reflecting the differences in physique and muscle strength attributable to gender and age.

The processing section 100 may also determine the exercise program based on the constitutional information in the user personal information. Constitutional information includes information regarding anamnesis, medications, and the like. Diabetes, for example, is known to cause a variety of complications. Depending on the type of complication and the progression thereof, it is necessary to avoid high-load exercise and proceed with exercise therapy carefully. Fundus hemorrhage and neurological damage have been known as the risks associated with exercise. In addition, since some patients may have diseases other than complications of diabetes, it is important to determine an exercise program in consideration of the respective diseases of the users. Also, if the patients are on insulin therapy, high-load exercise may cause hypoglycemia. Therefore, the exercise program is preferably determined according to the medications.

The processing section 100 may also determine the exercise program based on the physical measurement information in the user personal information. For example, by referring to height, weight, or BMI, it is possible to determine an exercise program that takes into account whether or not the physique of the user is suitable for the exercise. Alternatively, by using fasting blood glucose level or HbA1C, an exercise program can be created according to the progression of diabetes. The exercise program may also be determined according to the degree of obesity, such as waist size, triglycerides, cholesterol level, or the like.

That is, the processing section 100 receives gender, age, constitutional information, and physical measurement information as an input, and outputs an exercise program. The processing section 100 may also output an exercise program using a trained model obtained by, for example, machine learning. For example, if a user has achieved weight loss or other benefits by performing exercises according to a predetermined exercise program, a learning process is performed with respect to the user personal information of the user based on the training data in which the exercise program performed by the user is given as the correct answer label. If there is a difference between the exercise program instructed and the exercise content actually performed, it is preferable to use the exercise content actually performed as the correct answer label. If sufficient effects were not obtained or the exercise even worsened complications and the like although the user performed an exercise according to a predetermined exercise program, the combination of the user personal information of the user and the exercise program performed by the user may be used as negative training data. The storage section 170 stores the trained model. The processing section 100 is capable of determining an exercise program suitable for the user by reading out the trained model and performing an inference process using the user personal information of the target user as input. When using the table data shown in FIG. 9, the correct answer labels in the learning process and the output in the inference process are, for example, information that specifies the row of the table data.

However, the method of determining the exercise program based on the user personal information is not limited to those using machine learning. For example, the storage section 170 may store a plurality of items of information in which the user personal information and exercise programs are associated with each other. The information items stored in the storage section 170 have been evidenced to be effective in exercise therapy. The processing section 100 may calculate the degree of similarity between the user personal information of the target user and the user personal information stored, and select an exercise program associated with the user personal information determined to have a high degree of similarity. The process of determining the exercise program based on the user personal information can be changed in various ways.

The user personal information may also include performance result information that indicates the results of exercise performed by the user. The exercise therapy of the present embodiment assumes, for example, performing the exercise about three times a week. Therefore, if the frequency of the exercise is lower than this, or if more than a predetermined period of time has passed after the previous exercise, the frequency of the exercise is considered insufficient. Therefore, the processing section 100 may improve the user's motivation to perform the exercise by presenting an exercise program with low-intensity exercises.

The processing section 100 may perform a process of determining the exercise program in stages based on the user personal information. For example, a specific resistance exercise content, such as "push-ups", may be selected first, and the rest of the exercise content to be included in the exercise program may be determined based on the selected resistance exercise content. More specifically, in the determination of the exercise program of the present embodiment, the resistance exercise content may be determined first, and the aerobic exercise content may be determined based on the determined resistance exercise content. For example, the type (exercise ID) of resistance exercise to be included in the exercise program is determined based on the performance result information of the user, and the other resistance exercise contents and an aerobic exercise content are determined by selecting the row containing the type of resistance exercise thus determined from the table data in FIG. 9. Alternatively, the aerobic exercise content may be selected first, and the resistance exercise contents may be determined based on the selected aerobic exercise content. In this way, it is possible to appropriately determine an exercise program including more preferential exercise contents for the user to perform. If there are no particular preferential exercise contents, the resistance exercise content and the aerobic exercise content may be selected together.

2.4.3 Determining Exercise Program Based on Other Information

Although the process of determining the exercise program based on the user personal information was described above, other information may be used in determining the exercise program.

For example, the exercise program may be determined based on input from another terminal device TMB. The another terminal device TMB is, for example, a terminal used by medical staff. As described above with reference to FIG. 3, medical staff can view the information indicating the performance result of exercise of the patients using another terminal device TMB. If the medical staff determines that the patient is not performing the appropriate exercise, they may operate to determine or modify the exercise program for the user, i.e., the patient. The operation may be, for example, selecting a predetermined row from the table data in FIG. 9, or changing a part of the exercise program already determined. In this operation, they can change the exercise type, the number of sets, the exercise duration, and the like.

Alternatively, the another terminal device TMB may be a terminal used by a system administrator of the exercise management system. The system administrator is responsible for, for example, managing accounts of medical staff, user accounts, and exercise information. The system administrators can collect evidence of exercise therapy for diabetic patients by using the exercise management system of the present embodiment and the like. Accordingly, they may update the resistance exercise information, the aerobic exercise information, and the table data in FIG. 9. The trained models or algorithms used for determining the exercise program based on the user personal information may also be updated. In this case, even if the exercise program has already been determined for some users, it is possible to reconsider the exercise program based on the updated information, thereby allowing those users to perform the more appropriate exercise. Thus, the processing section 100 may perform a process of determining the exercise program based on updated information input by the system administrator using the another terminal device TMB.

Some users may desire to achieve weight loss or other results in a given exercise period. For example, if the user plans to see a doctor on a certain date, and if the user can achieve the results before the date, he/she can make a smooth decision regarding the future treatment. For example, the achievement may lead to favorable results for the user, such as reducing the prescribed medication or reducing the frequency of hospital visits. In this case, the user inputs the period from the present to the date of the hospital visit as the exercise period in the user terminal device TMA. The processing section 100 performs a process of determining an exercise program by which the user can achieve the results to some extent during the exercise period. For example, it is possible to select an exercise program that requires an exercise with higher intensity, compared with the case where the exercise period is not specified. However, since excessive frequency and intensity of exercise may damage the health of the user, it is desirable to set upper limits of frequency and intensity. The upper limit of the intensity is the upper limit of the reference value of the number-of-times threshold or the like.

The user may also specify the number of times of the exercise, instead of the exercise period. More specifically, the number of times of the exercise corresponds to the number of times the user performed the exercise program. Also in this case, for example, the exercise program is selected so that some results can be achieved by performing the specified number of times of the exercise. In addition, although the example in which the user inputs the number of times or the period of exercise is described above, the number of times or the period of exercise may be input by medical staff using another terminal device TMB.

2.5 Exercise Instructions Based on Exercise Content and Acquisition of Performance Result Information

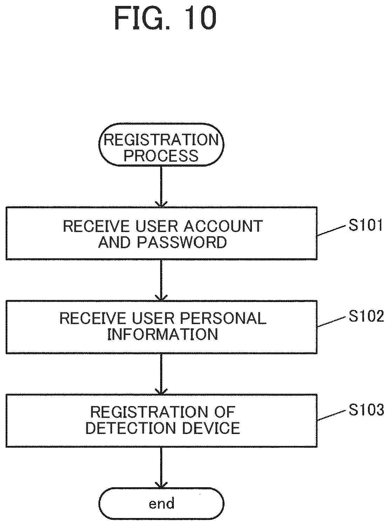

FIG. 10 is a flowchart showing a registration process required when the user uses the exercise management system for the first time. The exercise management system receives inputs of a user account and a password by the user (S101). For example, the user enters a user account and a password in the user terminal device TMA, and the server system 500 obtains and accumulates the user account and the password via the network 510. Next, the exercise management system receives an input of the user personal information (S 102). The information to be entered during the registration process includes gender, age, constitutional information, and physical measurement information. The constitutional information and the physical measurement information are manually input by the user, for example, by viewing prescriptions, diagnosis records, test results, and other information. However, if these items of information are stored as electronic data, the exercise management system may acquire and accumulate the user personal information by acquiring the corresponding electronic data. The exercise management system also performs a registration process for the detection device 600 owned by the user (S 103). For example, exercise management system also performs a process of connecting (pairing) the user terminal device TMA with the detection device 600. When the user wears a plurality of detection devices 600, the information to differentiate and manage the plurality of detection devices 600 is registered.

Similarly, the registration process is performed also for medical staff who use the exercise management system for the first time. In the registration process of medical staff, the exercise management system creates, for example, accounts and passwords of the medical staff, and also obtains and accumulates information of the medical staff. The information of the medical staff includes, for example, the names, workplaces, and the like of the medical staff. A process of associating the medical staff with the user (patient) is also necessary. For example, in the registration process shown in FIG. 10, the user (patient) inputs information for identifying the medical staff in charge of the user. It is also possible to allow the medical staff to input information for identifying the patient. The process of associating medical staff with patients can be changed in various ways.

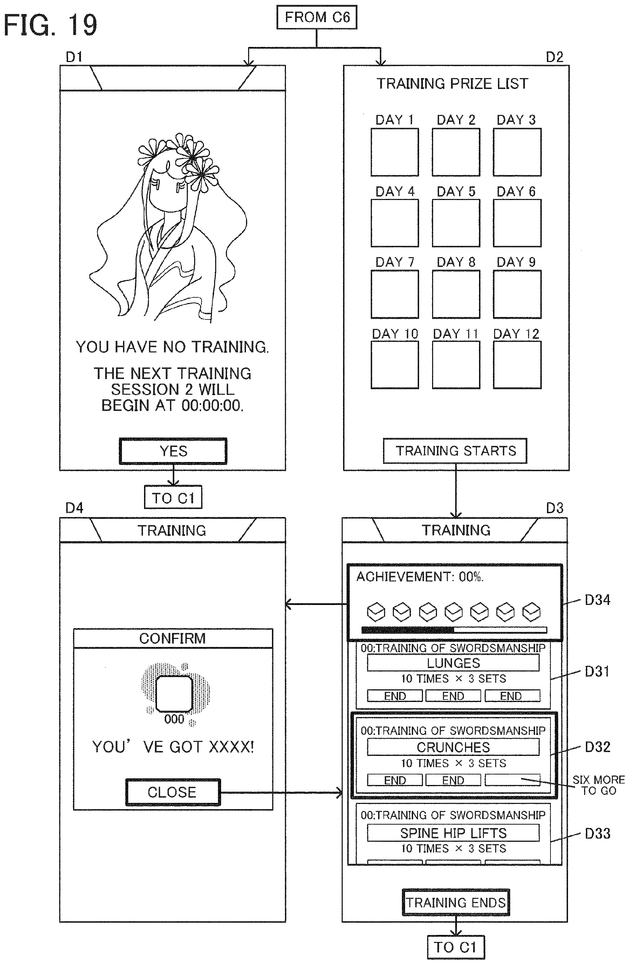

FIG. 11 is a flowchart explaining the processes in the exercise management system when the user performs exercises. First, the exercise management system processes the login of the user (S201). Specifically, it accepts the user account and the password input by the user and performs an authentication process. After the login attempt by the user is processed, the exercise management system performs a process of presenting an exercise program that the user is supposed to perform next (S202). The process of S202 is performed to, for example, display the resistance exercise content and the aerobic exercise content included in the exercise program in the display section 190 of the user terminal device TMA, and make the user to select. The display of the resistance exercise content is the display of text, the number-of-times threshold, and the number of sets corresponding to the exercise item (name), for example, as described later with reference to D31 to D33 in FIG. 19. The exercise management system accepts a selection operation by the user in response to the presentation process of S202 (S203).

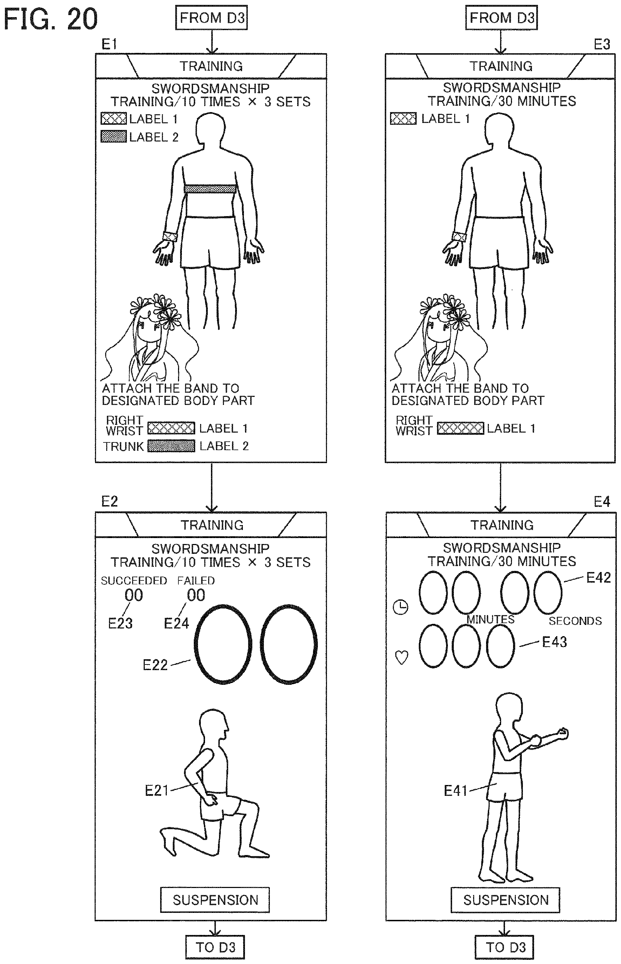

When the operation to select the resistance exercise is received (Yes in S204), the exercise management system instructs to attach the detection device 600 to a part according to the selected type of resistance exercise (S205). The instructions to wear the detection device 600 may be a process of displaying an image on the display section 190 of the user terminal device TMA, and/or a process of outputting sounds. The process of S205 is performed to display a screen, which is described later with reference to E1 in FIG. 20, for example. The exercise management system then starts acquiring motion information from the detection device 600 (S206).

The exercise management system also performs a presentation process to explain the specific body movements (S207). The process of S207 may be performed by displaying an image in the display section of the user terminal device TMA, and/or outputting sounds. For example, the processing section 100 displays the images shown in FIG. 15A to FIG. 16B and E2 in FIG. 20. The process of S207 may be performed before the exercise, or it may be continued until the exercise ends. For example, as described later with reference to E2 in FIG. 20, the number of times of motions in the resistance exercise and the results of depth determination and form determination can be presented continuously during the exercise to encourage the user to exercise appropriately.

When the resistance exercise is started by the user, the exercise management system performs a process of counting the number of times of motions (S208), and upon counting up, the depth determination (S209), the form determination (S210), and the completion determination (S211) are performed.

FIG. 12 is an explanatory view of a process of counting the number of times of motions. The horizontal axis in FIG. 12 represents time while the vertical axis represents motion information. The motion information refers to information of the posture angle that represents the angle of the detection device 600 with respect to the reference direction. The motion information to be counted is the information obtained from the detection device 600 attached to the major moving parts in the resistance exercise. For example, in the case of push-ups, the counting process is performed based on the motion information obtained from the detection device 600 attached to the upper arm.

In the counting process, first, when an angle change of more than or equal to a predetermined degree is detected, it is determined as motion detection (A1). After detecting the motion, counting-up is started when an angle change of more than or equal to a predetermined degree in the reverse direction is detected (A2). Also in the second time onwards, A3 corresponds to motion detection and A4 corresponds to counting-up. For example, in the case of push-ups, counting-up is started when the elbow is bent at 20° or more and then extended at 20° or more. In this way, the process of counting the number of times of motions is performed based on whether or not a given part moves to a certain extent or more. In the case of a resistance exercise in which the left and right legs are moved alternately, such as lunges or side lunges, the detection device 600 for depth determination may be attached to both the left and right legs so as to count the number of times of motions one at each time. Alternatively, the detection device 600 may be attached to one of the feet. In this case, the number of times of motions is counted up once for a single motion of the left leg and a single motion of the right leg. The processing section 100 may obtain the number of times of motions by doubling the count-up results. Alternatively, the number-of-times threshold may be set for the information indicating the number of times of motions of one of the legs in advance.

When the counting-up is performed, determination as to whether the single motion is appropriate as a resistance exercise is performed based on the depth threshold and the form threshold. As described above with reference to FIG. 6, the part to which the detection device 600 is attached is fixed according to the type of resistance exercise. Further, the information as to whether the evaluation angle to be compared with the depth threshold or the form threshold is an angle with respect to the horizontal direction or width of angle change has also been known. Therefore, by performing a comparison process according to the type of resistance exercise, it is determined whether the depth and the form satisfy the criteria, respectively.

FIGS. 13A to 13C are diagrams for showing the depth determination and the form determination in push-ups. In FIGS. 13A to 13C, the horizontal axis represents time, and the vertical axis represents the angle of the detection device 600 with respect to the reference direction. As mentioned above, in push-ups, the detection device 600 is attached to an upper arm and the chest. Since the depth determination is performed on the condition that the elbow bends more than or equal to a predetermined angle, a process of comparing the change range (angle change) of the motion information from the detection device 600 attached to the upper arm with the depth threshold (e.g., 80°) is performed. Since the form determination is performed on the condition that the waist is not inwardly curved, a process of comparing the change range (angle change) of the motion information from the detection device 600 attached to the chest with the form threshold (e.g., 40°) is performed.

FIG. 13A is an example of the motion information when proper push-ups are performed, wherein $\theta$ represents an angle indicating the moving range of the upper arm. In the example shown in FIG. 13A, $\theta \geq 80°$ is satisfied; therefore, it is determined that the push-ups were performed at an appropriate depth. $\varphi$ is an angle indicating the moving range of the chest. In the example shown in FIG. 13A, $\varphi \leq 40°$ is satisfied; therefore, it is determined that the push-ups were performed with a proper form.

In the example shown in FIG. 13B, the angle change $\theta$ of the arm is $\theta < 80°$. Therefore, although the number of times of motions is counted up, the elbow is not bent at a sufficient angle; accordingly, it is determined that the depth is insufficient. In the example shown in FIG. 13C, the angle change $\varphi$ of the chest is $\varphi > 40°$. This means that the chest greatly swings and the waist is inwardly curved; therefore, it is determined that the form is inappropriate. The same applies to resistance exercises other than push-ups. Based on the comparison between the motion information from the detection device 600, and the depth and form thresholds, the processes of S209 and S210 are performed.

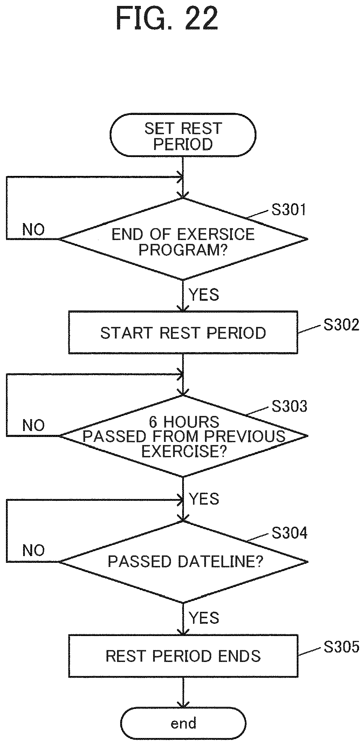

In S211, the exercise management system determines whether or not the number of times of motions after counting-up has reached or exceeded the number-of-times threshold. If the number of times of motions exceeded the number-of-times threshold, the exercise management system determines that the target resistance exercise has been completed and the sequence returns to S202. When all of the resistance and aerobic exercises in the exercise program have been completed, the exercise management system displays the completion in S202. Specifically, the exercise management system may display a rest period to the start of the next exercise program, as described later with reference to FIG. 22. If there are any exercises undone, the exercise management system displays a screen in which the user can select and input the exercise undone.

As described above with reference to FIG. 9, the number of sets may be set for the resistance exercise.

For example, if two or more sets of the exercise are performed, the exercise management system counts up the number of sets performed once the number of times of motions exceeds the number-of-times threshold in S211. If the number of sets performed has not reached the specified number of sets, a process of instructing the user to rest during the interval is performed, and then the sequence returns to S208 to continue the resistance exercise. When the number of sets performed reaches the specified number of sets, the exercise management system determines that the target resistance exercise has been completed, and the sequence returns to S202.

When an operation to select an aerobic exercise is received (No in S204), the processing section 100 gives an instruction to attach the detection device 600 to a predetermined part (S212). For example, in aerobic exercises, the detection device 600 is attached to the wrist; however, the detection device 600 may be attached to other parts. Also, although one detection device 600 is sufficient during aerobic exercise, using two or more detection devices 600 is not excluded. After the instruction to attach the detection device 600, the processing section 100 starts acquiring vital information from the detection device 600 (S213).

The processing section 100 starts measuring the exercise time at the timing when the operation to start aerobic exercise is received (S214). The aerobic exercise may be a combination of a plurality of motions, such as steps, punches, and the like as described above. Since it is difficult for users to memorize these motions, it is desirable for the processing section 100 to specifically present the motions of aerobic exercise (S215). The processing section 100 also detects the heart rate as vital information. Since the method of calculating the heart rate based on outputs of a photoelectric sensor has been known, the detailed explanation thereof is omitted. The exercise management system may display the current heart rate on the screen for the instructions of motions, as described later with reference to E4 in FIG. 20. By displaying the heart rate, the user can appropriately adjust the load of the aerobic exercise, such as the extent of the movement.

Next, the processing section 100 determines the completion of the aerobic exercise (S216). Specifically, the determination of the completion of aerobic exercise is made according to whether or not the exercise time has reached a given set value (e.g., 30 minutes). Until the exercise is continued to the point of the set value (No in S216), the above process is continued. When the exercise time reaches the set value (Yes in S216), the processing section 100 determines that the performance of aerobic exercise is complete and the sequence returns to S202.

Performing the processes shown in FIG. 11 makes it possible to provide appropriate exercise instructions to the user and obtain the motion information and the vital information during exercise. The processing section 100 performs a process of storing the performance result information based on the acquired motion information and vital information in the storage section 170 while associating it with the user.

For example, the processing section 100 performs a process of accumulating the results of counting the number of times of motions obtained based on the motion information, as well as the results of the depth determination and the form determination. For example, the total number of times of motions of the resistance exercise, the number of times of motions that satisfied both the depth and form criteria, the number of times of motions that satisfied the depth criteria but did not satisfy the form criteria, the number of times of motions that satisfied the form criteria but did not satisfy the depth criteria, and the number of times of motions that did not satisfy both the depth and form criteria are accumulated. Alternatively, it is also possible to obtain a resistance exercise score based on the above respective numbers of times of motions, and store the resistance exercise score. The resistance exercise score is, for example, numerical information that increases with an increase in counts of exercise performed with the appropriate depth and form.

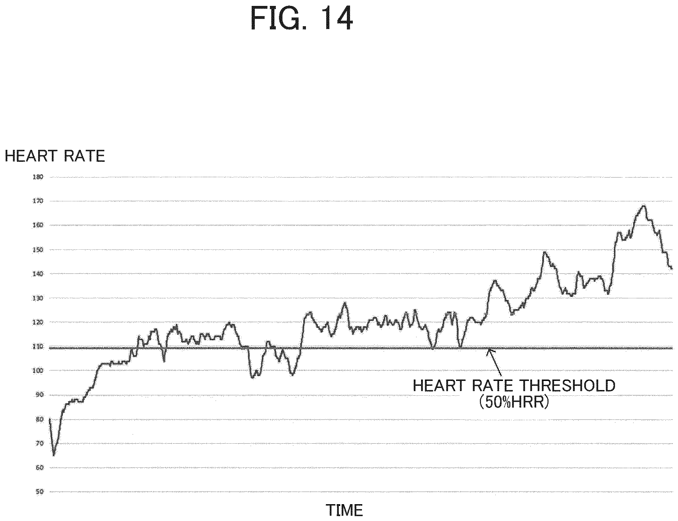

The exercise management system also accumulates information of heart rate, which is vital information obtained based on the sensor output. The information of heart rate is, for example, time-series variation of the heart rate, as shown in FIG. 14. By storing the time-series heart rates, it is possible to clearly present changes in heart rate during the exercise or the relationship thereof with the heart rate threshold. The exercise management system may also accumulate an average heart rate and the maximum heart rate during the aerobic exercise, or comparison results showing whether or not the average heart rate and the maximum heart rate exceed the heart rate threshold. Alternatively, it is possible to obtain an aerobic exercise score based on the time the aerobic exercise was continued or the results of comparison of the average heart rate and the maximum heart rate with the heart rate threshold, and store the aerobic exercise score.

The exercise management system may also perform a process of giving a prize to the user when the user completes the exercise. The prize is, for example, a discount ticket for the gym. For example, the exercise management system may send a serial number, a barcode, or other information to the user terminal device TMA. Then the user is given a discount for the gym by presenting the information using the user terminal device TMA. Alternatively, it is possible to store the address of the user as the user personal information, and send the prize by mail. In this case, the exercise management system performs a mailing process. The prize given to the user may also be electronic data used in the application. For example, items to be used in the game may be given as a prize. The details of the prizes in the game are described later.

2.6 Combination of Exercise and Game

The exercise management system of the present embodiment may perform a game process based on the performance result information. The game process may be a process of executing a first game associated with exercise and a second game that is not associated with exercise. By setting a rest period, the users can be prevented from excessive exercise. It is also possible to assign a plurality of games or a plurality of game effects to a single type of exercise. In this way, the users can enjoy different games and game effects for the same type of exercise. When the first and second games are played as described above, the game herein means the first game.

The users may be given prizes based on the performance result information of the exercise in the game. In this case, a prize that can be used in the second game may be given based on the performance result information of the first game. Thus, the effects of the prize obtained by the exercise are maintained. Furthermore, the game effects assigned to the exercise in the first game or the first game itself assigned to the exercise may be changed according to the state of use of the prize in the second game. This increases the user's interest in both the first and second games.

The details of the game process according to the present embodiment are described below.

2.6.1 Overview of Combination

In exercise therapy, it is important to make the users continue the exercise for a long period of time, for example, at least several months. However, users who need exercise therapy, such as a type-2 diabetic patient or a type-2 pre-diabetic patient, presumably do not have exercise habits and have low motivation for continuous exercise. Therefore, in the present embodiment, exercise may be combined with a game so as to improve the user's motivation to exercise.

The game implemented by the exercise management system according to the present embodiment may be a single-player game or a multiplayer game. The game is also not limited to specific types and may be applied to various games, such as an action game, an RPG, a fighting game, a shooting game, a competition game, a rhythm game, a puzzle game, or the like. In the case of a battle game, the battle may be a one-to-one battle or a many-to-many battle. The battle may also be a one-to-many (one versus a plurality of enemies) battle or a many-to-one (plurality of allies versus an enemy) battle. The opponent may be another user or an NPC (Non Player Character). The game may also be performed by forming a party of a plurality of cooperating users to battle against an NPC enemy. In addition, the game may be arranged such that the user does not operate the character by himself/herself; instead, AI (Artificial Intelligence) operates the character of the user. In this case, all or some of the characters of the user may be operated by AI.

The game processing section 104 of the exercise management system performs a game process based on the motion information and the vital information acquired by the detection device 600 while the user performs the exercise. The game processing section 104 performs processing of the game played by the user and generates game effects for game objects. For example, the game processing section 104 performs a process of advancing the game played by the user, and when the conditions for generating game effects are satisfied, the game processing section 104 performs a process of generating game effects for game objects. The game objects are objects that appear in the game image generated by the game process. For example, the game image is composed of images of a plurality of game objects. The game objects are objects of characters, backgrounds, or items that appear in the game. A character object is an object of a moving body that moves around the game space, such as a person, a robot, a monster, an animal, a car, a train, an airplane, or a ship. The background objects are objects that represent maps (terrain), buildings, courses (roads), mazes, spectator stands, mountains, trees, walls, water surfaces and the like. The item objects are objects that represent tools, treasure chests, weapons, protections, equipment, or parts used in the game. The items are used to activate special effects in the game, increase the attack or defense power of a character, restore the character's physical strength, or strengthen or evolve the character.

The game effects are used to change the game parameters used for the game process, generate game events, or change the game situation of the user. The game parameters are set for game objects, such as characters, and are parameters of offensive power, defensive power, durability, hit points, magic points, or levels. An event is, for example, an event that occurs in relation to a game object, such as an event that diverges or changes the game progress. The game status includes the game score status, the game level status, the gameplay status, the game progress status of the user, the game map/environment status, or the statuses of characters used by the user in the game.

The display processing section 120 performs a process of displaying a game screen in which the images of game objects are shown. For example, the display processing section 120 performs a process of displaying a game screen in the display section 190. The game screen is a screen used by the user to play the game. For example, during the gameplay by the user, a game image composed of images of a plurality of game objects is displayed in the display section 190 as a game screen.

In the present embodiment, performing exercise is one of the conditions for game progress. Therefore, it is possible to make the user continue the exercise by the motivation to advance the game. In addition, prizes may be given in the game on the condition that the user committed the exercise. In this way, the acquisition of prizes will also serve as motivation, thus it is possible to lead the user to perform a continuous exercise.

An example of a game or game effects according to the exercise, and the flow of the game process including giving prizes is described below.

2.6.2 Association of Game or Game Effects with Exercise

For example, in the case of combining RPG and push-ups, the game can be played by changing the objects of the characters based on the motion information, thereby performing game effects of avoiding traps in a dungeon. The game effects refer to the way of presenting the game status or the like to the user and information to determine the way of presentation. For example, the game effects are information that determines the type, the number, and the locations of the objects in the game screen. Specifically, when the performance result information is acquired as a result of the exercise by the user, the game effects are generated based on the performance result information, and the game situation changes. The game effects of the present embodiment are, in the narrow sense, the information for determining the objects to be displayed at the time. The type of exercise and the game effects may be associated one-to-one. In the above example, if push-ups are performed, the game effects are limited to those of avoiding traps.

However, in order to have the user perform the exercise continuously, it is important to keep the user amused. Even if exercise and a game are combined to increase motivation, if the game effects have no variation, the effect of increasing motivation may diminish as the exercise is repeated. Therefore, in the present embodiment, a plurality of game effects are assigned to a single type of exercise. In this way, even if the same type of exercise is performed, different game effects may be performed, thus preventing the user from getting bored and encouraging the user to perform a continuous exercise.

FIGS. 15A and 15B illustrate two examples of game effects associated with push-ups, which are, for example, examples of game screen displayed in the display section 190. In the examples of FIGS. 15A and 15B, the reception section 102 obtains the motion information from the detection device 600. The processing section 100 determines the number of push-up motions, the depth, and the form of the push-ups based on the motion information, as described above with reference to FIG. 12 and FIGS. 13A to 13C.

In the example shown in FIG. 15A, the game processing section 104 performs the game effects in which the character avoids traps by updating the object OB1 of the character and the trap object OB2 when counting-up is detected. In the example shown in FIG. 15B, the game processing section 104 updates the object OB3 of the character when counting-up is detected, thereby performing game effects in which the character looks down the cliff. In the example shown in FIG. 15B, a friendly monster object OB4 may be placed under the cliff, followed by an update process of the object.

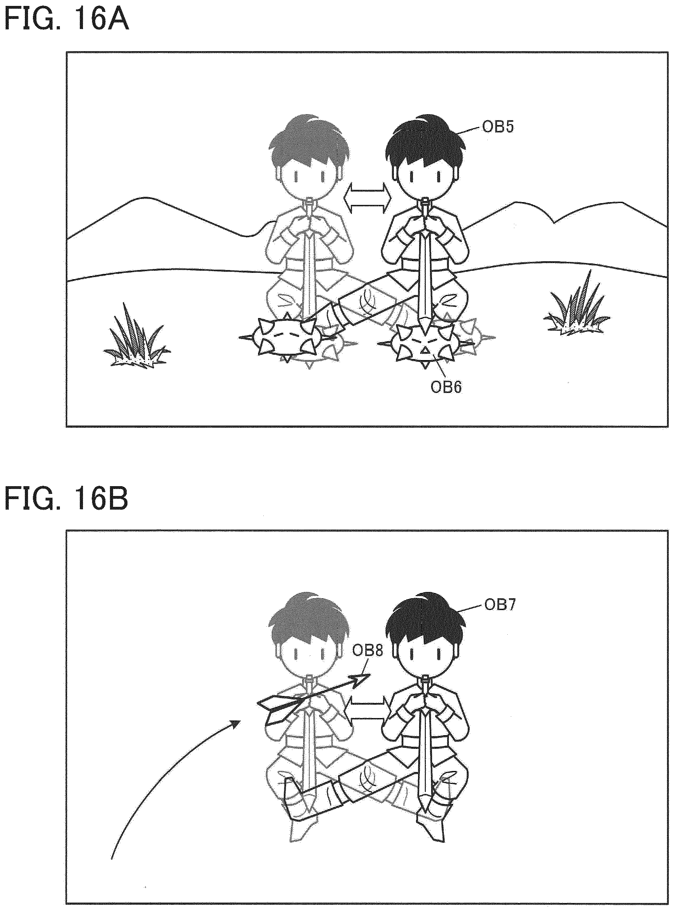

FIGS. 16A and 16B illustrate two examples of game effects associated with side lunges, which are, for example, examples of game screen displayed in the display section 190. In the examples shown in FIGS. 16A and 16B, the processing section 100 determines the number of times of side lunge motions, the depth, and the form based on the motion information.

In the example shown in FIG. 16A, the game processing section 104 performs game effects in which the character defeats an enemy by updating the object OB5 of the character and the monster object OB6 as the enemy when counting-up is detected. For example, by placing proliferating monsters or a large number of monster objects, it is possible to naturally perform game effects of attacking monsters in succession. In the example shown in FIG. 15B, the game processing section 104 performs game effects in which the character avoids attacks by updating the object OB7 of the character and the offensive object OB8 (e.g., arrow, magic, etc.) to attack the character when counting-up is detected.

Although FIGS. 15A to 16B illustrate examples of game effects associated with push-ups and side lunges, it is possible to associate a plurality of game effects also with other resistance exercises. The number of game effects that can be associated with a single resistance exercise is not limited to two, but can be three or more.

Although the association of resistance exercise with game effects is described above, the same method can be used for aerobic exercise. For example, if the aerobic exercise is a set of a plurality of motions such as steps, arm curls, punches, and the like, a plurality of game effects can be associated with each of the steps or other motions.

In the above, examples in which a plurality of game effects are assigned to a single type of exercise are described. For example, in the examples shown in FIGS. 15A to 16B, an RPG is played, and the game effects in the RPG game are switched according to the situation. However, the method of the present embodiment is not limited to this, and a plurality of games may be assigned to a single type of exercise.

For example, the game processing section 104 of the present embodiment may be capable of performing a game process for a plurality of games among various games, such as, as described above, an action game, an RPG, a fighting game, a shooting game, a competition game, a rhythm game, a puzzle game, and the like. For example, the game processing section 104 associates an action game and an RPG with push-ups. The game processing section 104 then switches, depending on the situation, whether to perform a game process to perform an action game based on the motion information when the user performs push-ups, or perform a game process to perform an RPG based on the motion information. In this way, it is possible to prevent the user from getting bored and encouraging the user to perform continuous exercise also by switching the game itself that is associated with the exercise.

In addition, it is also possible that a plurality of games are associated with a single type of exercise, and that a plurality of game effects are associated with each other in a single game. For example, the first and second game effects, which are game effects for an action game, and the third and fourth game effects, which are game effects for an RPG, may be associated with push-ups.

The game or game effects to be selected from among the plurality of games or game effects are determined based on the user personal information, for example. For example, the game processing section 104 stores the selection history of games or game effects as the user personal information, and gives priority to games or game effects that have been selected fewer times. It is also possible to perform a process of prioritizing a game(s) or game effects that have a history of being properly executed by the user.

The processing section 100 may also perform a process of determining a goal to complete in the game process, in addition to the selection of the game or game effects. The goal to complete is information by which the determination as to whether or not to advance the game can be made. More specifically, in games involving exercises, the goal to complete is information based on exercise intensity. For example, the number-of-times threshold, the depth threshold, and the form threshold are set as the goal to complete, and the game is advanced when the performance result information exceeds the goal. However, the specific goal to complete can be changed in various ways, and it is possible to use, for example, only the number-of-times threshold as the goal to complete. As is clear from above, the goal to complete is information that is determined when an exercise to be performed by the user is determined, depending on the exercise.

### 2.6.3 First and Second Games

In exercise therapy, it is not desirable to make users exercise excessively, as it may damage their health. Therefore, it is preferable to have a rest period of a certain length after a single exercise program is performed. During the rest period, the user needs to refrain from exercises, and playing games involving exercises is also not desirable. However, if the user is not allowed to advance the game during the rest period, the user may lose motivation to perform the game and may also lose motivation to exercise. Therefore, the games of the present embodiment may include the first game that involves exercise and the second game that does not involve exercise. In this way, the game can be continued while the user is not performing exercises, thus appropriately maintaining the user's motivation to exercise. The following specifically explains the flows of the first game and the second game.

FIG. 17 is a diagram for a game cycle of the present embodiment. In the following, an example of a game having an objective to acquire and strengthen a character capable of battles and to win the battles. Battles using characters can be played in various ways, including simulation games, RPG, shooting games, and fighting games.

In the method of the present embodiment, the user can obtain prizes in the game by performing exercises, as shown in B1 and B2 in FIG. 17. B1 and B2 correspond to the first game involving exercises, and a game process based on the motion information and the vital information is performed, for example, as shown in FIGS. 15A to 16B. The prize herein may be a character, equipment to strengthen the character, or an item. The contents of the prizes, the timing of giving the prizes, and the timing of use of the prizes can be changed in various ways. The details thereof are described later.

The user then uses the prize(s) acquired in the first game to strengthen the fighting strength (B3). Specifically, the user performs an operation to, for example, equip his/her character with weapons and/or protections, or use level-up items. A battle game is then played with the strengthened character (B4). Since the prizes are also given by completing the battle game (B5), the user further strengthens the fighting strength using the acquired prize (B3). B3 to B5 correspond to the second game, which does not involve exercises.

In the example shown in FIG. 17, the fighting strength is increased by performing exercises, thereby allowing the user to dominate the battle game. Therefore, if the user is interested in the second game, the user is expected to also be interested in the first game that involves exercises, thereby it is possible to make the user actively perform exercises. In other words, the game can be used to motivate users to perform exercises.

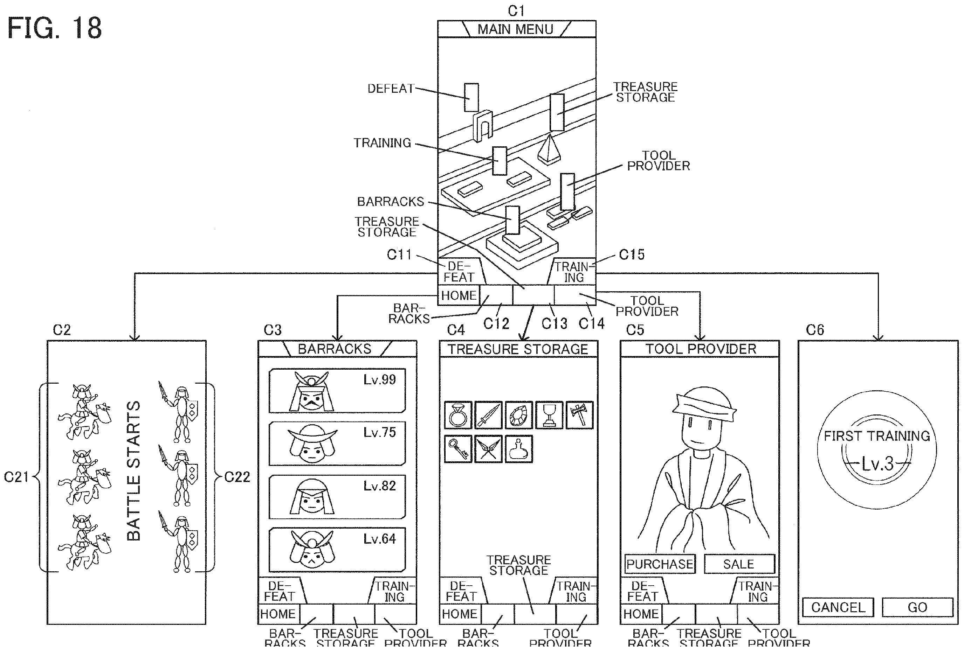

FIGS. 18 to 20 are diagrams showing an example of screen transitions in the games of the present embodiment, including the first game and the second game. FIG. 18 illustrates a home screen of a game and shows screen transitions from the home screen. The home screen shown in C1 is a screen suitable for the transitions to the menus in the game, and corresponds to, for example, the screen that appears first after the user logged in to the game. On the home screen, five menus can be selected: "defeat," "barracks," "treasure storage" "tool provider," and "training". For example, the menus can be selected by selecting the corresponding areas shown in C11 to C15.

C2 is an example of the defeat screen that is displayed when "defeat" is selected. The defeat screen is used to start the battle game in FIG. 17. For example, in the "defeat" screen, ally characters C21 and enemy characters C22 are displayed, and the battle game is started by accepting the user's command input. Although it is not shown in FIG. 18, a screen for selecting characters to be used for the battle may be displayed between the C1 and C2 screens.

C3 is an example of a character list screen that is displayed when "barracks" is selected. The character list screen shows the levels and face graphics of the characters acquired by the user. When the operation to select a character is received, the display moves to a power-up screen (not shown). In the power-up screen, change of character's equipment, training of characters to a higher level or the like, recovery of characters using items, and the like are performed. In other words, the "barracks" and related screens are used to strengthen the fighting strength as shown in FIG. 17.

C4 is an example of an item list screen that is displayed when "treasure storage" is selected. The item list screen shows the items acquired by the user. When an operation to select an item is received in the item list screen, the display may move to the power-up screen described above.

C5 is an example of an item trading screen that is displayed when "tool provider" is selected. In the item trading screen, the user can buy or sell items using the currency set in the game.

C6 is a training screen that is displayed when "training" is selected. The "training" herein corresponds to the first game, which involves exercises.

FIGS. 19 and 20 are diagrams showing screen transitions in the first game. When the user presses the "GO" button in C6 of FIG. 18, the game processing section 104 determines whether or not the user is in the resting period. The method of specifically setting the rest period is described later. If the user is in the rest period, the game processing section 104 displays the screen shown in D1 of FIG. 19. In D1, the start time of the next exercise, i.e., the end of the rest period, is displayed. At this time, by displaying an NPC with communication events, the user's frustration at not being able to perform the first game involving exercises can be suppressed.

If it is not in the rest period, the game processing section 104 displays the screen shown in D2 of FIG. 19. D2 is a bonus screen for giving game prizes based on the number of times (days) of exercises. The screen in D2 may be displayed, for example, at the start of an exercise program, and may be omitted in other situations.

When an operation to select the training start button in D2 is performed, the game processing section 104 displays the screen shown in D3. D3 displays items D31 to D33 showing the resistance exercise contents and the aerobic exercise contents included in a single exercise program, and item D34 showing the prizes to be given by performing the exercise program. In FIG. 19, three resistance exercise contents of the exercise program are displayed; however, other resistance exercise contents and aerobic exercise contents can be shown as the user scrolls the screen. FIG. 19 shows an example using prizes that are given at the start of the exercise program (shown in the screen in D2) and prizes that are given according to the performance status of each exercise included in the exercise program (shown in D34). When an operation to select D34 is performed, the game processing section 104 displays a screen specifically showing the prizes that have been given, as shown in D4. If an operation to select the close button in D4 is performed, the game processing section 104 operates to return to the screen display in D3.

When an operation to select any of the resistance exercise contents D31 to D33 is performed, the game processing section 104 performs a process of displaying a screen of exercise instructions. The order of performing the exercise may be set for each exercise content included in a single exercise program. In this case, a screen is displayed in which only the exercise content that is supposed to be performed next can be selected from among a plurality of exercise contents and other exercise contents cannot be selected.

When an operation to select a resistance exercise content is performed, the game processing section 104 displays the screen shown in E1 of FIG. 20. E1 is a screen showing the parts to which the two detection devices 600 are attached. In E1, an image and character strings that clearly indicate the parts to which the detection devices 600 are attached are displayed. It is also possible to display an NPC similar to that in E1 in FIG. 19 and perform effects in which the NPC supports the "training".

For example, when the reception section 102 starts acquiring the motion information from the detection device 600, the game processing section 104 operates to move to the exercise instruction screen in E2. In E2, an image describing motions in lunges (E21) and the measured number of times of the motions (E22) are displayed. In addition, the number of successful motions (E23) and the number of unsuccessful motions (E24) are displayed based on the depth determination and the form determination. For example, the processing section 100 determines success when both depth and form satisfy the conditions, and determines failure when at least one of the conditions is not satisfied. When a preset number of times of motions of resistance exercises have been performed, the game processing section 104 performs a process of transition to the screen shown in D3 of FIG. 19.

When an operation to select an aerobic exercise content is performed in the screen D3, the game processing section 104 displays the screen shown in E3. Similarly to E1, E3 is a screen showing the part to which the detection device 600 is attached. However, in aerobic exercises, it is sufficient that the vital information, which is, in the narrow sense, information regarding heart rates, is acquired. Therefore, the user may be instructed to wear only one detection device 600.

For example, when the reception section 102 starts acquiring the vital information from the detection device 600, the game processing section 104 operates to move to the exercise instruction screen in E4. In E4, an image (E41) for showing the specific motions (punch, etc.) to be performed in aerobic exercise, the time elapsed since the start of the aerobic exercise (E42), and the current heart rate (E43) are displayed. When the preset time has elapsed, the game processing section 104 performs a process of transition to the screen shown in D3 of FIG. 19.

When an operation to select the suspension button in E2 or E4 is performed, the game processing section 104 performs a process to suspend the resistance exercise or the aerobic exercise. The details of the suspension process are described later with reference to FIG. 23.

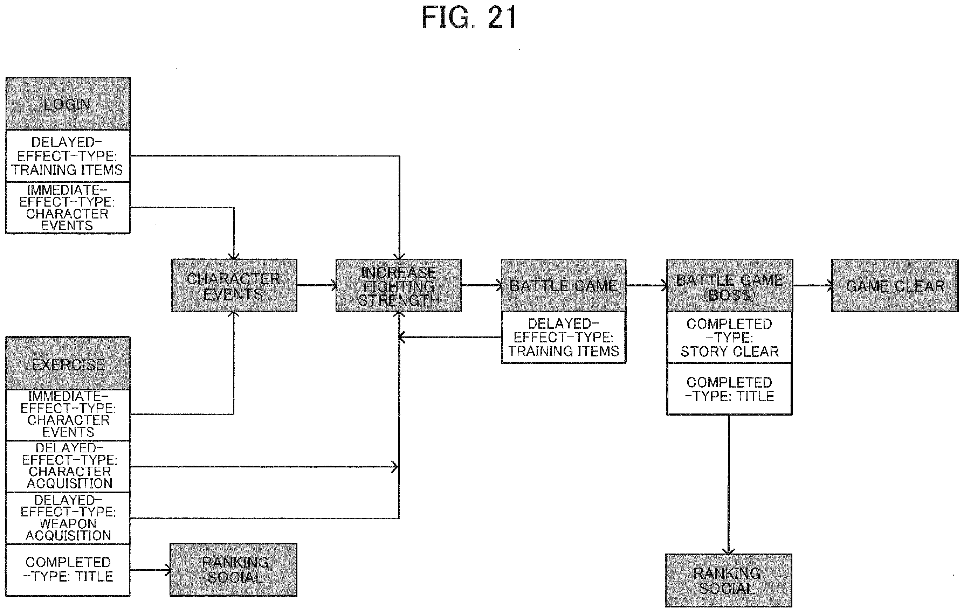

FIG. 21 shows an example of the types of prizes and the timing of giving the prizes in the first game and the second game. The prizes of the present embodiment include three types: immediate effect, delayed effect, and completed prize. The immediate-effect-type prizes make the user feel some effect immediately after he/she acquired the prize. Examples of the immediate-effect-type prizes include a prize that generates an event, such as the progress of a sub-story involving the NPC. The delayed-effect-type prizes are, for example, characters to be used for battles, weapons allowed for the characters to use, and items to train the characters. The delayed-effect-type prizes make the user feel effects at stages of character strengthening, completion of a battle game, or the like, after the user obtained the prize. The completed prizes are prizes for which the acquisition thereof itself is the goal, such as a title that can be used in a game community using SNS.

As shown in FIG. 21, the game of the present embodiment allows the user to obtain login prizes based on login, exercise prizes based on exercise, and battle prizes based on battle games. Login prizes are given for logging in to a game application. Login prizes, for example, include training items and NPC events. The exercise prize may be a prize for starting the exercise program as described above with reference to D2 in FIG. 19, and/or a prize for performing the exercises contained in the exercise program as described above with reference to D34 and D4. The exercise prizes include NPC events, characters, weapons, and titles. The battle prizes are given when the user satisfies a certain condition, such as defeating a predetermined enemy in a battle. The battles may be classified into normal battles and boss battles, and the prizes to be given may vary depending on the content of the battle. The battle prizes include training items and titles.

As shown in FIG. 21, by appropriately giving immediate-effect-type prizes, delayed-effect-type prizes, and completed prizes using various conditions, it is possible to have the user feel the effects of the prizes in many situations in the game cycle. More specifically, by providing a plurality of types of prizes for exercise with different characteristics, i.e., the immediate-effect-type prizes, delayed-effect-type prizes, and completed prizes, it is possible to have the user feel the effects of the prizes at both early and late timings after the user completed the exercise. This makes it possible to maintain their motivation to exercise.

As described above, in this embodiment, a plurality of games or game effects are associated with a single exercise. The games or game effects to be used in the game process are determined based on the user personal information, for example. However, the method of selecting the games or game effects is not limited to this method, and the games or game effects may be selected based on the second game. For example, as shown in FIG. 21, prizes that can be used in the second game are given based on the performance results of the first game. The game processing section 104 of the present embodiment may select a game or game effects based on the acquisition status and the use status of the prize in the second game.

For example, if a weapon for a character to use is given as a prize based on the first game, the game or game effects are changed based on the state of equipping weapons. For example, if a first game effect enabling an attack with a sword and a second game effect enabling an attack with a spear are both available as the game effects, the first game effect is performed by default, and the second game effect is performed on the condition that the spear given as a prize is equipped. In this way, the first game is changed when the prize(s) obtained in the first game is used in the second game; as a result, the first game and the second game can be more strongly connected. This can increase the motivation of the user to play the first game, thereby it is possible to have the user continuously perform the exercise.

2.6.4 Rest Period and Suspension of Exercise

The rest periods and suspension are described below. FIG. 22 is a flowchart showing a process of determining the rest period. When the process is started, the processing section 100 determines whether or not all performances of the exercises contained in a single exercise program have been completed (S301). The completion of the performances herein may include forced termination, which is described later. If the performances in the exercise program have not been completed (No in S301), the sequence returns to S301 and the determination continues.

When the performances in the exercise program have been completed (Yes in S301), the rest period is started (S302). After setting the rest period, the processing section 100 determines whether or not the time elapsed since the timing of the last exercise is 6 hours or more (S303). The 6 hours is merely an example, and a different length of time may be set. If the exercise program is successfully completed, the timing of the last exercise is the same as the timing of completion of the exercise program. In contrast, if the exercise program was forced to terminate, the timing of the last exercise is the timing earlier than the timing of the completion of the exercise program. If 6 hours have not elapsed (No in S303), the sequence returns to S303 and the determination continues.

If 6 hours have passed (Yes in S303), it is determined whether or not the current time has crossed the dateline (S304). The dateline is, for example, 3:00 a.m., but other timings such as 0:00 a.m. may be set. The dateline may also be changed according to the user's lifestyle. If the dateline has not been crossed (No in S304), the sequence returns to S304 and the determination continues.

If the dateline has been crossed (Yes in S304), the processing section 100 determines that the rest period has ended (S305). The rest period is a period from S302 to S305 wherein the start of the first game is prohibited. After the process of S305, the user is allowed to perform the next exercise program; therefore, the processes shown in FIG. 22 are performed again for the next exercise program.

In the present embodiment, as shown in FIG. 22, the rest period continues until a given length of time (6 hours) has elapsed after the last exercise and also until the dateline has been crossed after the completion of the last exercise. In this way, it is possible to prevent the user from performing a plurality of exercise programs in a short period of time, thereby suppressing the load of the exercise.

As explained above, the rest period is set when a single exercise program is completed, that is, when both the resistance exercise and the aerobic exercise are completed. However, a rest period for the resistance exercise and a rest period the for aerobic exercise may be set separately. More specifically, the rest period may be set when the resistance exercise is completed or when the aerobic exercise is completed. If the exercise is forcibly terminated, the rest period is set based on the exercise performed at a later timing among the resistance exercise and aerobic exercise.

Further, it may be necessary to suspend the exercise during the performance of a single exercise program due to unavoidable reasons such as using a restroom, eating, or the like. If suspension is not allowed the whole time, it may decrease the convenience of the user. On the other hand, if the suspension of the exercise is unlimitedly allowed, a single exercise program takes a long time, which is not appropriate for exercise therapy.

Therefore, in the present embodiment, the period and the timing of the forced termination of the exercise program are set. For example, if 48 hours have passed since the start of the exercise included in the exercise program, the exercise program is forcibly terminated. The reference timing for the forced termination may be a different timing, such as when the exercise is suspended. The time length of 48 hours is also an example. The forced termination may be executed based on a different length of time.

If the exercise program is forcibly terminated, the progress of the exercise program as of the timing may be discarded, and the same exercise program may be instructed to perform again. Alternatively, when the exercise program is forcibly terminated, the progress of the exercise program as of the timing may be stored, and the next exercise program may be instructed to perform. In this case, the record indicating that the exercise program that was forcibly terminated was not completed can be stored. By allowing forced termination, it is possible to prevent setting of an excessively long suspension.

The suspension of resistance exercise is managed in units of sets, for example. FIG. 23 is a flowchart explaining processes in a single set. As described above, the process of determining whether or not to forcibly terminate the exercise program is performed apart from the flowchart in FIG. 23. If the determination to forcibly terminate the exercise program is made, the sequence in FIG. 23 ends right away. When a single set of the resistance exercise is started, the processing section 100 determines whether or not the set has been successfully completed (S401). Specifically, the determination of S401 is the determination as to whether or not the number of times of motions has reached the number-of-times threshold. If Yes in S401, the set finishes successfully. If No in S401, the processing section 100 determines whether or not to suspend the resistance exercise (S402). Specifically, S402 is the determination as to whether or not the suspension button has been operated in E2 of FIG. 20. If No in S402, the sequence returns to S401 and the process continues.

If the suspension operation is performed (Yes in S402), the processing section 100 determines whether or not the suspension in the present set is three or more times (S403). If Yes in S403, the processing section 100 performs abnormal termination of the set. More specifically, information such as the number of times of motions as of the timing is stored. If the suspension is the first or second time (No in S403), the processing section 100 waits for the reception of the restart operation (S404), and when the restart operation is performed (Yes in S404), the sequence returns to S401 and the process continues. By thus switching the process according to the number of suspensions, it is possible to ensure both the convenience of the user and the effectiveness of exercise therapy.

The suspension related to aerobic exercises can be assumed in the same way as in FIG. 23. In this case, the determination of normal termination in S401 is replaced by the determination as to whether or not the predetermined exercise time has elapsed. In addition, considering the fact that aerobic exercise is performed for a relatively long time (e.g., 30 minutes), the allowable number of times of suspension may be set to a larger value than that in resistance exercise. For example, in S403, determination as to whether or not the suspension has been performed for four or more times is performed.

Also, in aerobic exercise, the vital information, such as the heart rate value, is important. If the suspension continued for a long time, the values of the vital information in the exercise state may return to the values in the normal state. Therefore, when aerobic exercise is suspended, instructions to perform preparatory exercise may be added at the time of suspension and at the time of restart. In this way, changes in vital information due to suspension can be suppressed. For suspension of aerobic exercise, the process may be performed separately for suspension of less than a predetermined duration and suspension for a predetermined duration or longer. For example, suspension of 2 minutes or less may be regarded as a pause; in this case, the user is allowed to smoothly return to the aerobic exercise being performed. For example, when the user returned from a pause, the preparatory exercise for restart is omitted. In contrast, for suspension of 2 minutes or longer, as described above, instructions to perform preparatory exercises are given to suppress the change in vital information. Further, it is also possible to continue the detection of the vital information during a period of 2 minutes or less after the suspension operation, and stop the detection of the vital information when 2 minutes or more have passed. The time of 2 minutes herein is merely an example, and can be changed as necessary considering the degree of change in vital information over time.

2.7 Types of Performance Result Information

In the present embodiment, it is possible to obtain a first performance result information based on the motion information, the vital information, and a first determination criteria, and obtain a second performance result information based on a second determination criteria, which is different from the first determination criteria. The first performance result information is used for the presentation on the user terminal device TMA. The presentation herein may be a game process that includes the display of a game screen, and the like. The second performance result information is used for the presentation on another terminal device TMB. This is more specifically described below.

As described above, in exercise therapy, it is important to continuously perform exercises of appropriate intensity. The intensity in the present embodiment refers to the number-of-times threshold, the depth threshold, the form threshold, and the like in resistance exercise, and the heart rate threshold, and the like in aerobic exercise. However, if the intensity is overly considered, it may become difficult to perform the exercise continuously.

For example, users who are extremely unskilled at exercises or who are highly obese may have difficulties in performing even low-intensity exercise. Therefore, even if the number-of-times threshold, the depth threshold, and the form threshold are set, for example, to the reference values as in the examples of FIGS. 6 and 7, such users may not be able to reach the number-of-times threshold of the motions or fail to perform the exercise with appropriate depth and form. In the case of combining exercise and games, even if these users attempt the exercise (or play the first game), the game may not be advanced or no prizes will be given. As the result of failure is repeated, the user's motivation to exercise may decrease and the user may not continue to perform the exercise.

Therefore, when the performance results are presented to these users, the processing section 100 may set the determination threshold to a value that can be more easily achieved, compared with the reference value. For example, in the case of push-ups, an exercise with an evidenced effectiveness as an exercise therapy can be performed by setting the depth threshold to 80° or higher. However, for users who are unskilled at exercises, the depth threshold is set to a smaller value. As a result, the depth is determined to be sufficient even if the bending of the elbow is small. Similarly, although the reference value of the form threshold is 40°, the form threshold is set to a greater value. As a result, the form is determined to be appropriate even if the waist is inwardly curved. Similarly, the number-of-times threshold may be set to a value smaller than the reference value, thereby allowing users to more easily perform exercises. In this case, the first game that involves exercise can be more easily advanced and the prizes can be easily obtained. This makes it possible to appropriately increase the motivation of the user to perform exercises.

The prize may be different when the exercise is performed according to the reference value and when the exercise is performed according to a value more easily achievable than the reference value. For example, although the prize may be given also when the exercise is performed according to a value lower than the reference value, the prize may have less variety or of a smaller number compared to the case where the exercise is performed according to the reference value. By doing so, since the users are believed to desire more prizes, it is possible to encourage the users who are unskilled at exercise to increase the intensity of exercise.

However, in view of medical staff who diagnoses, treats, and guides type-2 diabetic patients or type-2 pre-diabetic patients, the determination results based on a determination threshold more easily achievable, compared with the reference value are not desirable. The reference value for each threshold in the present embodiment is an evidence-based value for certain effects such as weight loss. Since it is unclear whether the users can obtain effects when they perform an exercise that deviates from the reference value, the exercise that deviates from the reference value should not be regarded as satisfying the appropriate number of motions, depth, or form of exercise. Therefore, it is desirable for the processing section 100 to set the determination threshold according to the reference value in the process of presenting the exercise performance results to medical staff.

As described above, by changing the determination threshold between the user and medical staff, it becomes possible to maintain the user's motivation to exercise while allowing the medical staff to perform a diagnosis based on medical evidence. In other words, in this embodiment, the determination criteria are changed between the process of displaying the exercise performance results in the user terminal device TMA and the process of displaying the exercise performance results in another terminal device TMB. The display of performance results in the user terminal device TMA is specifically a game process of displaying a game screen. In other words, the transition of the game screen and the execution of the game effects are performed based on the processes using a relatively easily-achievable determination threshold.

FIG. 24 illustrates an example of a screen displayed in another terminal device TMB. The processing section 100 scores the resistance exercise and the aerobic exercise based on their determination thresholds both according to the reference value, and displays the results. For example, as shown in FIG. 24, the date on which the exercise was performed is displayed in a calendar while associating it with the overall exercise performance results on the same date (F1). Further, when any date in the calendar is selected, the results of the resistance exercise and the aerobic exercises performed on the selected date are displayed (F2).

The score of the resistance exercise is determined based on the number of times of motions, the depth, and the form. For example, the score is 100 (◎) when all of the number of times of motions, the depth, and the form satisfy the required conditions, and the score is 0 (-) when the exercise is not performed. For example, if the exercise program is forcibly terminated in the middle of the program, the subsequent resistance exercise(s) is regarded as not having been performed. If at least one of the required conditions of the number of times of motions, the depth, and the form, is not satisfied, the score varies within a range greater than 0 and less than 100. For example, if the number of times of motions is less than the number-of-times threshold, the score is 25 (x). Even though the number of times of motions reached the number-of-times threshold, if more than half of the motions performed had inappropriate depth and form, the score is 50 (△). Further, even if the depth and the form were insufficient in some motions, if more than half of them satisfied the required conditions, the score is 75 (○).

The score of aerobic exercise is determined based on the exercise duration and heart rate. For example, the score is 0 if the exercise is not performed, the score is 25 if the exercise duration is less than the specified time (e.g., 30 minutes), the score is 50 if the exercise duration satisfies the specified time but both the average heart rate and the maximum heart rate are less than the heart rate threshold, the score is 75 if the exercise duration satisfies the specified time and the maximum heart rate is equal to or more than the heart rate threshold, and the score is 100 if the exercise duration satisfies the specified time and both the average heart rate and the maximum heart rate are equal to or more than the heart rate threshold.

The process of scoring the resistance exercise and the aerobic exercise is not limited to the example shown above, and can be changed in various ways. For example, the score may be determined in smaller units, for example, on a 1-point basis.

FIG. 24 also has a graph showing long-term time-series change of the performance results (F3). The graph shows the results of the resistance exercise in the form of a bar chart herein, and shows the results of the aerobic exercise in the form of a line chart.

Medical staff is often responsible for a large number of patients. Therefore, if the performance result information is overly detailed, it is not desirable for him/her because it takes time to understand the information. In contrast, In FIG. 24, the exercise performance results are quantified and displayed with graphics and graphs such as ○ and ×. Therefore, it is possible to present the general performance results in a simple manner. In addition, since the performance results for a month or longer are displayed on a single screen, it is easy to grasp whether or not the appropriate exercise has been performed continuously.

FIG. 25 illustrates an example of a screen displayed in the user terminal device TMA. As shown in FIG. 25, a screen different from the game screen may be used to present the performance results to the user. In FIG. 25, the resistance exercise and the aerobic exercise are rated in 3 scales, i.e., "○," "△," and "×"; however, as shown in FIG. 24, the results may be rated in 5 scales or rated with finer details. In this case, the score may be calculated based on determination criteria more easily achievable, compared with the reference value, as described above. Since the screen shown in FIG. 25 does not include game-specific information such as characters, weapons, items, and the like, it is possible to display the exercise performance results with high browsability.

2.8 Friend User

The exercise management system of the present embodiment may also perform a process of presenting results of a comparison with other users in order to increase the user's motivation to exercise. For example, the processing section 100 performs a process of setting a friend user. A friend user herein means another user who has performed or will perform the same level of exercise as that of the target user. For example, the processing section 100 performs a process of setting another user, who started the application of the present embodiment (game application in the narrow sense) at the same time as a given user and having user personal information highly similar to that of the given user, as a friend user. As described above, since the exercise program is determined based on the user personal information, the another user is expected to perform similar exercise contents at a similar pace as the target user. The setting of a friend user is not limited to this method, and may be performed based on the similarity of the generated exercise program or the similarity of the performance result information. The processing section 100 may also set a friend user based on an input by the user. For example, it is possible to select a friend user from among people the user actually knows, such as colleagues at work, or from among SNS friends.

FIG. 26 shows an example of a screen displaying the difference between the performance result information of a given user and the performance result information of a friend user. The example in FIG. 26 shows the difference in performance result information by displaying a graph showing the number of times the exercise program was performed. Thus displaying the difference enables the user to recognize that the degree of accomplishment with respect to the exercise instructions and the degree of the progress of the exercise therapy are behind compared with those of the friend user. FIG. 26 clearly shows that the number of times the user performed the exercise program is smaller than that of the friend user. Since this will encourage the user to compete with other users, it is possible to increase the user's motivation to exercise compared to the case where the difference is not used. Although an example of displaying the difference in the number of times the user performed the exercise program has been described above, the specific display example can be changed in various ways, for example, the difference in intensity may be displayed.

The exercise management system of the present embodiment may also provide a service to add comments to the performance result information and post it on SNS. The processing section 100 may also perform a process of displaying the comments posted by a friend user on the screen displaying the difference. In the example shown in FIG. 26, the display shows a comment "I lost XX kg", which reports a weight loss, posted by a friend user A. By displaying such information, users viewing the screen in FIG. 26 would expect that the result of weight loss can be obtained if they perform the exercise program to the same extent as that of the friend user A. This can further improve the user's motivation to exercise. In the case where the effects of performing exercises are thus displayed, the target friend users are not limited to those who started using the game program at the same time as that of the user. For example, the friend user may be another user who started using the game program earlier than the target user. In this case, the fact that the level of exercise achievement of the target user is lower than that of the friend user does not matter, and the display regarding the friend user is used to illustrate future goals and expected effects.

However, such a display showing the difference with a friend user who has a higher achievement is not desirable for users who are sufficiently performing exercises. The user who has been sufficiently performing exercises refers to, for example, a user who has been continuously performing exercises three times a week, or a user who has been performing exercises with the intensity specified by the exercise instructions. If such users are encouraged to perform more intense exercise, there is a risk of damaging the user's health contrary to intentions. Therefore, the processing section 100 may omit the display of the difference from the friend user when it is determined that the degree of achievement for the exercise instructions of the target user is high based on the performance result information of the user, in other words, when it is determined that the target user has been performing exercises with sufficient frequency and intensity.

Alternatively, if the processing section 100 determines that the degree of achievement for the exercise instructions is high based on the performance result information of the user, the processing section 100 may suggest the user to reduce exercises by displaying the difference between the user and the friend user who has a lower degree of achievement for the exercise instructions compared with the user. A friend user herein means another user who is determined to be having been performing exercises at a standard frequency and intensity, for example.

FIG. 27 shows another example of a screen displaying the difference between a given user's performance result information and a friend user's performance result information. This is an example similar to FIG. 26 and displays a graph showing the number of times of performances in exercise programs. In this case, it is clearly shown that the exercise load of the user is higher than that of the friend user. This makes it possible to prevent the user from excessively performing exercises. Alternatively, as shown in FIG. 27, the processing section 100 may display, for example, text that suggests reducing exercises, such as "slow down the pace of exercise".

As described above, it is possible to control the exercise of the user according to the situation by switching between the process of encouraging the user to perform exercises using the difference, the process of omitting the display of the difference, and the process of reducing exercises using the difference, based on the performance result information of the user.

In the above, an example in which the friend user is another user has been described. However, the friend user herein may be a virtual user generated by the processing section 100. For example, the processing section 100 uses the performance result information of a plurality of users accumulated in the storage section 170 and the exercise program of the target user to estimate the reference data, which is the standard performance result information of the exercise program. The estimated reference data is then used as the performance result information of the friend user, and the difference between the performance result information of the user and the reference data is displayed. Alternatively, the processing section 100 may generate reference data based on information regarding an exercise therapy with evidence-based efficacy or information regarding evidence-based harm to health, and use the reference data as the performance result information of the virtual user. The processing section 100 may also generate different types of reference data based on age, gender, or the like. The reference data is not limited to those determined each time. The reference data associated with each exercise content may be stored in advance in the storage section 170. In this case, the friend users are not limited to real users; therefore, it is possible to set friend users suitable for comparison. This makes it possible to appropriately perform promotion and suppression of exercise.

By setting a virtual user as a friend user, it is possible to encourage the users to compare and compete with others. However, in the case of displaying the reference data, the process of treating the reference data as performance result information of a virtual user (friend user) is not indispensable. For example, the reference data may be used for the display of the difference without being associated with a specific user. Although the effects of encouraging competition with others are smaller than the case of using a friend user, the use of difference makes it possible to present to the user in a simple manner whether or not the user is performing exercises with an appropriate load.

Although the present embodiments have been described above in detail, those skilled in the art will readily appreciate that many modifications are possible in the embodiment without materially departing from the novel teachings and advantages of the present disclosure. Accordingly, all such modifications are intended to be included within the scope of the present disclosure. For example, in the specification or the drawings, each of terms that is at least once used together with another broader-sense or synonymous term can be replaced with the other term at any part of the specification or the drawings. In addition, the reception process, the game process, the display process, and the like are also not limited to those described in the present embodiment, and methods equivalent to those are also included in the scope of the present disclosure.

Reference Numerals

100: Processing section, 102: Reception section, 104: Game processing section, 112: Management section, 120: Display processing section, 130: Sound processing section, 160: Operation section, 170: Storage section, 172: Game information storage section, 174: Exercise information storage section, 176: User personal information storage section, 180: Information storage medium, 190: Display section, 192: Sound output section, 192: Communication section, 194: I/F section, 195: Portable information storage medium, 196: Communication section, 500: Server system, 510: Network, 600: Detection device, 610: Motion detection section, 620: Vital detection section, OB1 to OB8: Object, TM, TM1 to TMn: Terminal device, TMA: User terminal device, TMB: Terminal device.

**Claims**

1. An exercise management system for managing exercise of a user, who is a type-2 diabetic patient or a type-2 pre-diabetic patient, the exercise management system comprising:

   a storage section for storing user personal information of the user;
   a processing section for determining, based on the user personal information, a resistance exercise content, which is information to specify a content of resistance exercise performed by the user, and an aerobic exercise content, which is information to specify a content of aerobic exercise performed by the user; and
   an acquisition section for acquiring motion information indicating motion of the user when exercise instructions are given based on the resistance exercise content, and vital information indicating vitals of the user when the exercise instructions are given based on the aerobic exercise content, wherein
   the processing section performs, based on the motion information and the vital information, a process of presenting performance result information indicating performance results of the resistance exercise and the aerobic exercise performed by the user on a user terminal device used by the user, or another terminal device.

2. The exercise management system as defined in claim 1, wherein the user personal information includes at least one of gender, age, constitutional information and physical measurement information of the user.

3. The exercise management system as defined in claim 1 or 2, wherein the user personal information includes the performance result information of at least one of the resistance exercise and the aerobic exercise performed by the user in the past.

4. The exercise management system as defined in any one of claims 1 to 3, wherein the resistance exercise content and the aerobic exercise content performed in a single exercise determined by the processing section is an exercise program including the resistance exercise content corresponding to a type of the resistance exercise selected from among a plurality of types of the resistance exercises, and the aerobic exercise content corresponding to a type of the aerobic exercise selected from among a plurality of types of the aerobic exercises.

5. The exercise management system as defined in claim 4, wherein the processing section determines a plurality of types of the exercise programs to be performed by the user in a given exercise period based on the user personal information.

6. The exercise management system as defined in any one of claims 1 to 5, wherein the processing section determines the resistance exercise content and the aerobic exercise content based on input from the another terminal device.

7. The exercise management system as defined in any one of claims 1 to 6, wherein the processing section determines the resistance exercise content and the aerobic exercise content based on an exercise period or a number of times of exercise when the exercise period or the number of times of exercise performed by the user is input from the user terminal device or the another terminal device.

8. The exercise management system as defined in any one of claims 1 to 7, wherein the storage section stores table data in which the resistance exercise content and the aerobic exercise content are associated with each other.

9. The exercise management system as defined in any one of claims 1 to 8, wherein, when one of the resistance exercise content and the aerobic exercise content is determined, the processing section determines the other one of the resistance exercise content and the aerobic exercise content based on the determined resistance exercise content or aerobic exercise content.

10. The exercise management system as defined in any one of claims 1 to 9, wherein the processing section determines a prize to be given to the user based on the performance result information of the user in response to the exercise instructions based on the resistance exercise content and the aerobic exercise content.

11. The exercise management system as defined in any one of claims 1 to 10, wherein the processing section performs a process of setting, as a friend user, another user or a virtual user who performs exercises corresponding to the resistance exercise content and the aerobic exercise content for which the user is instructed to perform.

12. The exercise management system as defined in claim 11, wherein the processing section performs a process of presenting information to encourage the user to exercise based on a difference between the performance result information of the user and the performance result information of the friend user.

13. The exercise management system as defined in claim 12, wherein, when the degree of achievement for the exercise instructions is determined to be higher than a given standard based on the performance result information of the user, the processing section performs a process of skipping presentation of the information to encourage the user to exercise based on the difference, or performs a process of presenting information suggesting that the user can reduce exercises based on the difference.

14. The exercise management system as defined in any one of claims 1 to 10, wherein the processing section

acquires reference data indicating a standard of the performance result information of the resistance exercise content and the aerobic exercise content for which the user is instructed to perform, and
performs a process of presenting information to encourage the user to exercise, or a process of presenting information that suggests that the user can reduce exercises, based on the difference between the performance result information of the user and the reference data.

15. The exercise management system as defined in any one of claims 1 to 14, wherein the processing section

presents a first performance result information obtained based on the motion information, the vital information, and a first determination criteria on the user terminal device, and
presents a second performance result information obtained based on the motion information, the vital information, and a second determination criteria, which is different from the first determination criteria, on the another terminal device.

16. The exercise management system as defined in any one of claims 1 to 15, further comprising

a motion detection section for detecting the motion information, and
a vital detection section for detecting the vital information,
wherein the motion detection section and the vital detection section are provided in a single wearable terminal device attached to the user.

17. A server system for managing exercises of a user, who is a type-2 diabetic patient or a type-2 pre-diabetic patient, the server system comprising:

a storage section for storing user personal information of the user;
a processing section for determining, based on the user personal information, a resistance exercise content, which is information to specify a content of resistance exercise performed by the user, and an aerobic exercise content, which is information to specify a content of aerobic exercise performed by the user; and

an acquisition section for acquiring motion information indicating motion of the user when exercise instructions are given based on the resistance exercise content, and vital information indicating vitals of the user when the exercise instructions are given based on the aerobic exercise content, wherein

the processing section performs, based on the motion information and the vital information, a process of presenting performance result information indicating performance results of the resistance exercise and the aerobic exercise performed by the user on a user terminal device used by the user, or another terminal device.

18. A terminal device for use in exercise management of a user, who is a type-2 diabetic patient or a type-2 pre-diabetic patient, the terminal device comprising:

a processing section for performing a first acquisition process to acquire a resistance exercise content and an aerobic exercise content determined based on user personal information of the user; an instruction process to give exercise instructions to the user based on the resistance exercise content and the aerobic exercise content; and a second acquisition process to acquire motion information indicating motion of the user when the exercise instructions are given based on the resistance exercise content, and vital information indicating vitals of the user when the exercise instructions are given based on the aerobic exercise content; and

a presentation section for presenting performance result information indicating performance results of resistance exercise and aerobic exercise performed by the user based on the motion information and the vital information.

19. An exercise management method for managing exercises of a user, who is a type-2 diabetic patient or a type-2 pre-diabetic patient, the exercise management method comprising:

performing a process of acquiring user personal information of the user;
performing a process of determining, based on the user personal information, a resistance exercise content, which is information to specify a content of resistance exercise performed by the user, and an aerobic exercise content, which is information to specify a content of aerobic exercise performed by the user;

performing a process of acquiring motion information indicating motion of the user when exercise instructions are given based on the resistance exercise content, and vital information indicating vitals of the user when the exercise instructions are given based on the aerobic exercise content; and

based on the motion information and the vital information, performing a process of presenting performance result information indicating performance results of the resistance exercise and the aerobic exercise performed by the user on a user terminal device used by the user, or another terminal device.

# FIG. 1A

SERVER SYSTEM ~500

~510

| TM1 | | TM2 | ... | TMn |

# FIG. 1B

# FIG. 1C

# FIG. 1D

# FIG. 1E

# FIG. 2

EP 4 008 413 A1

# FIG. 3

500

TMA

600(610,620)

TMB

EP 4 008 413 A1

# FIG. 4

DATE OF PREVIOUS
EXAMINATION AT
CLINIC/HOSPITAL

PERFORMANCE
RESULT
INFORMATION

USER PERSONAL INFORMATION

| NAME | GENDER | AGE | | TIME OF ONSET | INSULIN INJECTION | ... | HEIGHT | WEIGHT | BLOOD GLUCOSE LEVEL | ... | | | ... |

CONSTITUTIONAL INFORMATION    PHYSICAL MEASUREMENT INFORMATION

# FIG. 5

| RESISTANCE EXERCISE | LOWER BODY<br><br>· SQUATS<br>· LUNGES<br>· SIDE LUNGES<br>· CALF RAISES<br>· BACK KICKS<br>· SPINE HIP LIFTS |
| --- | --- |
| | UPPER BODY<br><br>· PUSH-UPS<br>· NARROW PUSH-UPS<br>· BACK EXTENSIONS<br>· LEG RAISES<br>· CRUNCHES<br>· PLANKS |

# FIG. 6

REFERENCE VALUE OF
NUMBER-OF-TIMES
THRESHOLD

| EXERCISE ID | EXERCISE ITEMS | |
|---|---|---|
| 1 | PUSH-UPS | 20 |
| 2 | NARROW PUSH-UPS | 20 |
| 3 | SQUATS | 20 |
| 4 | LUNGES | 20 |
| 5 | SIDE LUNGES | 20 |
| 6 | BACK EXTENSIONS | 10 |
| 7 | LEG RAISES | 20 |
| 8 | CRUNCHES | 15 |
| 9 | PLANKS | 30 |
| 10 | CALF RAISES | 20 |
| 11 | BACK KICKS | 20 |
| 12 | SPINE HIP LIFTS | 20 |

# FIG. 7

| EXERCISE ITEMS | DEPTH /FORM | BAND POSITION | EVALUATION ANGLE | REFERENCE VALUE |
|---|---|---|---|---|
| PUSH-UPS | DEPTH | UPPER ARM | RANGE OF MOTION OF UPPER ARM | 80° ≦ |
| | FORM | CHEST (BACK SIDE) | ANGLE CHANGE OF CHEST | ≦40° |
| NARROW PUSH-UPS | DEPTH | UPPER ARM | RANGE OF MOTION OF UPPER ARM | 70° ≦ |
| | FORM | CHEST (BACK SIDE) | ANGLE CHANGE OF CHEST | ≦50° |
| SQUATS | DEPTH | THIGH | RANGE OF MOTION OF THIGH | 80° ≦ |
| | FORM | CHEST (BACK SIDE) | RANGE OF MOTION OF CHEST | ≦70° |
| LUNGES | DEPTH | RIGHT THIGH, LEFT THIGH | ANGLE BETWEEN RIGHT THIGH AND GROUND | -10° ≦ |
| | FORM | CHEST (BACK SIDE) | ANGLE CHANGE OF CHEST | ≦30° |
| SIDE LUNGES | DEPTH | RIGHT THIGH, LEFT THIGH | ANGLE BETWEEN RIGHT THIGH AND GROUND | -20° ≦ |
| | FORM | RIGHT SHIN, LEFT SHIN | ANGLE DIFFERENCE BETWEEN LEFT THIGH AND LEFT KNEE WHEN RIGHT THIGH IS MAXIMALLY BENT (AND VICE VERSA) | ≦10° |
| LEG RAISES | DEPTH | THIGH | ANGLE BETWEEN THIGH AND GROUND (MAXIMUM VALUE) | 70° ≦ |
| | FORM 1 | THIGH | ANGLE BETWEEN THIGH AND GROUND (MINIMUM VALUE) | ≦10° |
| | FORM 2 | THIGH | ANGLE DIFFERENCE BETWEEN THIGH AND SHIN | ≦30° |
| BACK EXTENSIONS | DEPTH | — | — | — |
| | FORM | CHEST (BACK SIDE) | WHEN A SMALL ANGLE CHANGE OCCURS, THE SCORE IS INCREASED AND THE CUMULATIVE SUM IS REGARDED AS DEGREE OF INSTABILITY. | SCORE ≦10 |

EP 4 008 413 A1

# FIG. 8

| EXERCISE ID | INTENSITY | HEART RATE THRESHOLD | MOTION CONTENTS |
|---|---|---|---|
| 21 | LOW | 40%~85%HRR | STEPS, LEG CURLS, PUNCHES... |
| 22 | MEDIUM | 50%~85%HRR | STEP + ARM CURLS, KNEE-UPS... |
| 23 | HIGH | 60%~85%HRR | STEP + ARM CURLS, HEEL-TOUCHES, KICKS... |

# FIG. 9

| RESISTANCE 1 | | | RESISTANCE 2 | | | RESISTANCE 3 | | | RESISTANCE 4 | | |
|---|---|---|---|---|---|---|---|---|---|---|---|
| EXERCISE ID | INTEN-SITY | NUMBER OF SETS | EXERCISE ID | INTEN-SITY | NUMBER OF SETS | EXERCISE ID | INTEN-SITY | NUMBER OF SETS | EXERCISE ID | INTEN-SITY | NUMBER OF SETS |
| 1 | LOW | 2 | 6 | LOW | 2 | 3 | LOW | 2 | 11 | LOW | 2 |
| 2 | LOW | 2 | 4 | LOW | 2 | 7 | LOW | 2 | 10 | LOW | 2 |
| 1 | LOW | 2 | 5 | LOW | 2 | 8 | LOW | 2 | 12 | LOW | 2 |
| 2 | LOW | 2 | 9 | LOW | 2 | 3 | LOW | 2 | 10 | LOW | 2 |
| 1 | LOW | 2 | 4 | LOW | 2 | 8 | LOW | 2 | 12 | LOW | 2 |
| 2 | LOW | 2 | 5 | LOW | 2 | 6 | LOW | 2 | 11 | LOW | 2 |
| 1 | LOW | 3 | 3 | LOW | 3 | 7 | LOW | 3 | 12 | LOW | 3 |
| 2 | LOW | 3 | 4 | LOW | 3 | 8 | LOW | 3 | 10 | LOW | 3 |
| 1 | LOW | 3 | 5 | LOW | 3 | 9 | LOW | 3 | 11 | LOW | 3 |
| 2 | LOW | 3 | 3 | LOW | 3 | 6 | LOW | 3 | 12 | LOW | 3 |
| 1 | LOW | 3 | 4 | LOW | 3 | 7 | LOW | 3 | 11 | LOW | 3 |
| 2 | LOW | 3 | 3 | LOW | 3 | 9 | LOW | 3 | 10 | LOW | 3 |
| 1 | MEDIUM | 3 | 6 | MEDIUM | 3 | 8 | MEDIUM | 3 | 3 | MEDIUM | 3 |
| 2 | MEDIUM | 3 | 9 | MEDIUM | 3 | 4 | MEDIUM | 3 | 10 | MEDIUM | 3 |
| 1 | MEDIUM | 3 | 6 | MEDIUM | 3 | 8 | MEDIUM | 3 | 5 | MEDIUM | 3 |
| 2 | MEDIUM | 3 | 9 | MEDIUM | 3 | 3 | MEDIUM | 3 | 10 | MEDIUM | 3 |
| 1 | MEDIUM | 3 | 6 | MEDIUM | 3 | 8 | MEDIUM | 3 | 4 | MEDIUM | 3 |
| 2 | MEDIUM | 3 | 9 | MEDIUM | 3 | 5 | MEDIUM | 3 | 10 | MEDIUM | 3 |

| RESISTANCE 5 | | | RESISTANCE 6 | | | AEROBIC EXERCISE | |
|---|---|---|---|---|---|---|---|
| EXERCISE ID | INTEN-SITY | NUMBER OF SETS | EXERCISE ID | INTEN-SITY | NUMBER OF SETS | EXERCISE ID | EXERCISE TIME |
| null | null | null | null | null | null | 21 | 30 |
| null | null | null | null | null | null | 21 | 30 |
| null | null | null | null | null | null | 21 | 30 |
| null | null | null | null | null | null | 21 | 30 |
| null | null | null | null | null | null | 21 | 30 |
| null | null | null | null | null | null | 21 | 30 |
| null | null | null | null | null | null | 22 | 30 |
| null | null | null | null | null | null | 22 | 30 |
| null | null | null | null | null | null | 22 | 30 |
| null | null | null | null | null | null | 22 | 30 |
| null | null | null | null | null | null | 22 | 30 |
| null | null | null | null | null | null | 22 | 30 |
| 12 | MEDIUM | 3 | 11 | MEDIUM | 3 | 23 | 30 |
| 12 | MEDIUM | 3 | 7 | MEDIUM | 3 | 23 | 30 |
| 12 | MEDIUM | 3 | 11 | MEDIUM | 3 | 23 | 30 |
| 11 | MEDIUM | 3 | 7 | MEDIUM | 3 | 23 | 30 |
| 12 | MEDIUM | 3 | 11 | MEDIUM | 3 | 23 | 30 |
| 12 | MEDIUM | 3 | 7 | MEDIUM | 3 | 23 | 30 |

# FIG. 10

REGISTRATION PROCESS

RECEIVE USER ACCOUNT AND PASSWORD — S101

RECEIVE USER PERSONAL INFORMATION — S102

REGISTRATION OF DETECTION DEVICE — S103

end

FIG. 11

```
                    ┌──────────────┐
                    │   PERFORM    │
                    │   EXERCISE   │
                    └──────┬───────┘
                           │
                    ┌──────▼───────┐
                    │    LOG-IN     │──── S201
                    └──────┬───────┘
        ┌──────────────────┼────────────────────────────────────┐
        │          ┌───────▼────────┐                            │
        │          │ PRESENT EXERCISE│──── S202                  │
        │          │    PROGRAM      │                            │
        │          └───────┬────────┘                            │
        │          ┌───────▼────────┐                            │
        │          │ RECEIVE SELECTION│──── S203                 │
        │          │   OPERATION     │                           │
        │          └───────┬────────┘                            │
        │              S204 │                                    │
        │            ◇──────┴──────◇                             │
        │           ╱   RECEIVE      ╲         NO                │
        │          ◇   RESISTANCE     ◇─────────────────┐       │
        │           ╲  EXERCISE?     ╱                    │       │
        │            ◇──────┬──────◇                      │       │
        │             YES   │                             │       │
        │   ┌───────────────▼────────┐    ┌───────────────▼────────┐
        │   │ INSTRUCTIONS TO ATTACH │    │ INSTRUCTIONS TO ATTACH │
        │   │   DETECTION DEVICE     │    │   DETECTION DEVICE     │
        │   └──────── S205 ──┬───────┘    └──────── S212 ──┬──────┘
        │   ┌────────────────▼───────┐    ┌────────────────▼──────┐
        │   │  START ACQUISITION OF  │    │  START ACQUISITION OF │
        │   │   MOTION INFORMATION   │    │   VITAL INFORMATION    │
        │   └──────── S206 ──┬───────┘    └──────── S213 ──┬──────┘
        │   ┌────────────────▼───────┐    ┌────────────────▼──────┐
        │   │    PRESENT MOTIONS     │    │   START MEASUREMENT   │
        │   │                         │    │   OF EXERCISE TIME    │
        │   └──────── S207 ──┬───────┘    └──────── S214 ──┬──────┘
        │   ┌────────────────▼───────┐    ┌────────────────▼──────┐
        │   │    DETERMINATION       │    │    PRESENT MOTIONS    │
        │   │     OF COUNTS          │    │                        │
        │   └──────── S208 ──┬───────┘    └──────── S215 ──┬──────┘
        │   ┌────────────────▼───────┐           S216 │
        │   │    DETERMINATION       │          ◇──────┴──────◇
        │   │     OF DEPTH           │         ╱     END        ╲  NO
        │   └──────── S209 ──┬───────┘         ◇───────────────◇──┐
        │   ┌────────────────▼───────┐          ╲             ╱   │
        │   │ DETERMINATION OF FORM  │           ◇─────┬─────◇   │
        │   └──────── S210 ──┬───────┘             YES │         │
        │              S211  │                         └─────────┘
        │            ◇───────┴──────◇
        │       NO  ╱      END       ╲
        └─────────◇─────────────────◇
                    ╲             ╱
                     ◇─────┬─────◇
                       YES │
```

# FIG. 12

# FIG. 13B

INSUFFICIENT DEPTH

RIGHT ARM

θ

POSTURE ANGLE[deg]

Elapsed Time[s]

# FIG. 13C

INAPPROPRIATE FORM

φ

CHEST

POSTURE ANGLE[deg]

Elapsed Time[s]

# FIG. 13A

PROPER EXERCISE

φ

CHEST

RIGHT ARM

θ

POSTURE ANGLE[deg]

Elapsed Time[s]

# FIG. 14

HEART RATE

TIME

**FIG. 15A**

**FIG. 15B**

FIG. 16A

FIG. 16B

# FIG. 17

FIG. 18

**C1 — MAIN MENU**

DEFEAT

TRAINING

BARRACKS

TREASURE STORAGE

TREASURE STORAGE

TOOL PROVIDER

C11 — DE-FEAT
C15 — TRAIN-ING
HOME
BAR-RACKS
C12  C13  C14
TOOL PROVIDER

**C2**

BATTLE STARTS

C21 { }  C22 { }

**C3 — BARRACKS**

Lv.99
Lv.75
Lv.82
Lv.64

DE-FEAT    TRAIN-ING
HOME
BAR-RACKS   TREASURE STORAGE   TOOL PROVIDER

**C4 — TREASURE STORAGE**

TREASURE STORAGE

DE-FEAT    TRAIN-ING
HOME
BAR-RACKS   TOOL PROVIDER

**C5 — TOOL PROVIDER**

PURCHASE    SALE

DE-FEAT    TRAIN-ING
HOME
BAR-RACKS   TREASURE STORAGE   TOOL PROVIDER

**C6**

FIRST TRAINING
Lv.3

CANCEL    GO

EP 4 008 413 A1

# FIG. 19

FROM C6

**D1**

YOU HAVE NO TRAINING.

THE NEXT TRAINING
SESSION 2 WILL
BEGIN AT 00:00:00.

YES

TO C1

**D2**

TRAINING PRIZE LIST

DAY 1   DAY 2   DAY 3

DAY 4   DAY 5   DAY 6

DAY 7   DAY 8   DAY 9

DAY 10   DAY 11   DAY 12

TRAINING STARTS

**D4**

TRAINING

CONFIRM

000

YOU' VE GOT XXXX!

CLOSE

**D3**

TRAINING

ACHIEVEMENT: 00%.

— D34

00:TRAINING OF SWORDSMANSHIP
LUNGES
10 TIMES × 3 SETS
END   END   END

— D31

00:TRAINING OF SWORDSMANSHIP
CRUNCHES
10 TIMES × 3 SETS
END   END

— D32

SIX MORE
TO GO

00:TRAINING OF SWORDSMANSHIP
SPINE HIP LIFTS
10 TIMES × 3 SETS

— D33

TRAINING ENDS

TO C1

# FIG. 20

FROM D3

FROM D3

E1

TRAINING

SWORDSMANSHIP
TRAINING/10 TIMES × 3 SETS
LABEL 1
LABEL 2

ATTACH THE BAND TO
DESIGNATED BODY PART

RIGHT
WRIST          LABEL 1
TRUNK          LABEL 2

E3

TRAINING

SWORDSMANSHIP
TRAINING/30 MINUTES
LABEL 1

ATTACH THE BAND TO
DESIGNATED BODY PART

RIGHT
WRIST          LABEL 1

E2

TRAINING

SWORDSMANSHIP
TRAINING/10 TIMES × 3 SETS

SUCCEEDED    FAILED
00              00
E23           E24

E22

E21

SUSPENSION

E4

TRAINING

SWORDSMANSHIP
TRAINING/30 MINUTES

E42

MINUTES      SECONDS

E43

E41

SUSPENSION

TO D3

TO D3

# FIG. 21

EP 4 008 413 A1

```
┌─────────────────┐
│     LOGIN       │
├─────────────────┤
│ DELAYED-        │
│ EFFECT-TYPE:    │
│ TRAINING ITEMS  │
├─────────────────┤
│ IMMEDIATE-      │
│ EFFECT-TYPE:    │
│ CHARACTER       │
│ EVENTS          │
└─────────────────┘
```

```
┌─────────────────┐
│    EXERCISE     │
├─────────────────┤
│ IMMEDIATE-      │
│ EFFECT-TYPE:    │
│ CHARACTER       │
│ EVENTS          │
├─────────────────┤
│ DELAYED-        │
│ EFFECT-TYPE:    │
│ CHARACTER       │
│ ACQUISITION     │
├─────────────────┤
│ DELAYED-        │
│ EFFECT-TYPE:    │
│ WEAPON          │
│ ACQUISITION     │
├─────────────────┤
│ COMPLETED       │
│ -TYPE: TITLE    │
└─────────────────┘
```

```
┌─────────────────┐
│   CHARACTER     │
│    EVENTS       │
└─────────────────┘
```

```
┌─────────────────┐
│   INCREASE      │
│   FIGHTING      │
│   STRENGTH      │
└─────────────────┘
```

```
┌─────────────────┐
│  BATTLE GAME    │
├─────────────────┤
│ DELAYED-        │
│ EFFECT-TYPE:    │
│ TRAINING ITEMS  │
└─────────────────┘
```

```
┌─────────────────┐
│  BATTLE GAME    │
│     (BOSS)      │
├─────────────────┤
│ COMPLETED       │
│ -TYPE:          │
│ STORY CLEAR     │
├─────────────────┤
│ COMPLETED       │
│ -TYPE: TITLE    │
└─────────────────┘
```

```
┌─────────────────┐
│   GAME CLEAR    │
└─────────────────┘
```

```
┌─────────────────┐
│   RANKING       │
│   SOCIAL        │
└─────────────────┘
```

```
┌─────────────────┐
│   RANKING       │
│   SOCIAL        │
└─────────────────┘
```

# FIG. 22

# FIG. 23

```
        ( SUSPENSION )
               │
               ▼
            ╱S401╲
          ╱NORMAL ╲  YES
         ╱  END?   ╲──────────────────┐
          ╲       ╱                   │
           ╲     ╱                    │
            ╲ NO╱                     │
               │                      │
               ▼                      │
            ╱S402╲                    │
      NO  ╱SUSPEND?╲                  │
    ◄─────╲        ╱                  │
    │      ╲      ╱                   │
    │       ╲YES ╱                    │
    │          │                      │
    │          ▼                      │
    │       ╱S403╲                    │
    │     ╱SUSPENDED 3╲  YES          │
    │    ╱TIMES OR MORE?╲────────┐    │
    │     ╲            ╱         │    │
    │      ╲          ╱          │    │
    │       ╲   NO   ╱◄──────┐   │    │
    │          │            │   │    │
    │          ▼            │   │    │
    │       ╱S404╲          │   │    │
    │ YES ╱ACCEPT ╲  NO     │   │    │
    └────╱RESTART? ╲────────┘   │    │
          ╲        ╱            │    │
           ╲      ╱             ▼    ▼
                          ( ABNORMAL )  ( NORMAL END )
                          (   END    )
```

# FIG. 24

# FIG. 25

PREVIOUS EXERCISE
DAY BEFORE YESTERDAY

NEXT
EXAMINATION DAY

FEB. 22, 2018.
MALE,
36 YEARS OLD

EXERCISE GOAL
FOR THIS WEEK
TWO MORE TO GO

○ OVERALL RATING
OF THIS WEEK

EXERCISE GRAPH OF THIS WEEK

FIRST OF
FOUR ROUNDS.
OCT. 24

FOURTH OF
FOUR ROUNDS.
OCT. 19

THIRD OF
THREE ROUNDS
OCT. 18

SECOND OF
THREE ROUNDS
OCT. 12

FIRST OF
THREE ROUNDS

FIG. 26

LET'S TRY A
LITTLE HARDER.

YOU

USER A

I LOST
XX KG!

FIG. 27

SLOW DOWN
YOUR EXERCISE
PACE.

YOU

USER A

<div align="center">

**INTERNATIONAL SEARCH REPORT**

</div>

| | International application No. |
|---|---|
| | PCT/JP2020/029499 |

**A. CLASSIFICATION OF SUBJECT MATTER**
A63B  71/06(2006.01)i;  G16H  20/30(2018.01)i;  A61B  5/00(2006.01)i;  A61B
5/11(2006.01)i;  A61B 5/145(2006.01)i; A61B 5/22(2006.01)i
FI:      G16H20/30; A63B71/06 J; A61B5/00 102C; A61B5/22 110; A61B5/145;
        A61B5/11 230

According to International Patent Classification (IPC) or to both national classification and IPC

**B. FIELDS SEARCHED**

Minimum documentation searched (classification system followed by classification symbols)
A63B71/06; A61B5/00; A61B5/11; A61B5/145; A61B5/22; G16H20/30

Documentation searched other than minimum documentation to the extent that such documents are included in the fields searched
Published examined utility model applications of Japan          1922–1996
Published unexamined utility model applications of Japan        1971–2020
Registered utility model specifications of Japan                1996–2020
Published registered utility model applications of Japan        1994–2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2006-263002 A (NIPPON TELEGRAPH AND TELEPHONE CORP.) 05 October 2006 (2006-10-05) paragraphs [0017]-[0091] | 1-10, 14-19 |
| A | paragraphs [0017]-[0091] | 11-13 |
| Y | JP 2002-32487 A (SEKI, Masahiro) 31 January 2002 (2002-01-31) paragraph [0016] | 1-10, 14-19 |
| Y | JP 2004-54591 A (INSTITUTE OF TSUKUBA LIAISON CO., LTD.) 19 February 2004 (2004-02-19) paragraphs [0025], [0026] | 4-10, 11-16 |
| Y | JP 2014-39805 A (YAMATAKE KK) 06 March 2014 (2014-03-06) paragraphs [0074], [0075] | 6-10, 14-16 |
| Y | JP 2018-106403 A (DAINIPPON PRINTING CO., LTD.) 05 July 2018 (2018-07-05) paragraph [0003] | 10, 14-16 |
| Y | JP 2007-115200 A (NEC CORP.) 10 May 2007 (2007-05-10) paragraph [0016] | 14-16 |

☐ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 October 2020 (13.10.2020) | 27 October 2020 (27.10.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/029499

| Patent Documents referred in the Report | Publication Date | Patent Family | Publication Date |
|---|---|---|---|
| JP 2006-263002 A | 05 Oct. 2006 | (Family: none) | |
| JP 2002-32487 A | 31 Jan. 2002 | (Family: none) | |
| JP 2004-54591 A | 19 Feb. 2004 | (Family: none) | |
| JP 2014-39805 A | 06 Mar. 2014 | (Family: none) | |
| JP 2018-106403 A | 05 Jul. 2018 | (Family: none) | |
| JP 2007-115200 A | 10 May 2007 | (Family: none) | |

Form PCT/ISA/210 (patent family annex) (January 2015)

63

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018201905 A